# EUROPEAN PATENT SPECIFICATION

(11) **EP 1 126 870 B1**
(45) Date of publication and mention of the grant of the patent: **08.09.2004**
(21) Application number: 99956559.1
(22) Date of filing: 26.10.1999
(51) Int. Cl.: A61K 38/19, A61K 48/00, A61L 27/54, A61L 29/16, A61L 33/06, A61P 9/10

(54) **USE OF VEGF-C OR VEGF-D GENE OR PROTEIN TO PREVENT RESTENOSIS**
VERWENDUNG VON VEGF-C UND VEGF-D GENEN ODER PROTEINEN ZUR VORBEUGUNG DER RESTENOSE
UTILISATION DU GENE OU DE LA PROTEINE VEGF-C OU VEGF-D POUR PREVENIR LA RESTENOSE

(30) Priority: 26.10.1998 US 105587 P
(43) Date of publication of application: 29.08.2001
(73) Proprietor: LUDWIG INSTITUTE FOR CANCER RESEARCH, New York, New York 10158 (US); Licentia Ltd., 00130 Helsinki (FI); Yla-Herttuala, Seppo, 70211 Kuopio (FI)
(72) Inventor: YLA-HERTTUALA, Seppo University of Kuopio, FIN-70211 Kuopio (FI); ALITALO, Kari Uni. of Helsinki, Haartman Inst., FIN-00014 Helsinki (FI); HILTUNEN, Mikko, O. University of Kuopio, FIN-70211 Kuopio (FI); JELTSCH, Markku M Uni. of Helsinki, Haartman Inst, FIN-00014 Helsinki (FI); ACHEN, Marc, G. Ludwig Institute for Cancer Res., Parkville, VIC 3050 (AU)
(74) Representative: Jump, Timothy John Simon
(86) International application number: PCT/US1999/024054
(87) International publication number: WO 2000/024412

(56) References cited:
- WO-A-98/07832
- WO-A-98/19712
- WO-A-98/33917
- WO-A-98/49300
- WITZENBICHLER, B. J. ET AL: "Bioactivity of vascular endothelial growth factor-2 (= VEGF - C ) in vitro and in vivo following intraarterial administration of recombinant protein or intravascular gene transfer in a rabbit ischaemic hindlimb model." EUROPEAN HEART JOURNAL, (1997) VOL. 18, NO. ABSTR. SUPPL., PP. 5. MEETING INFO.: XIXTH CONGRESS OF THE EUROPEAN SOCIETY OF CARDIOLOGY TOGETHER WITH THE 32ND ANNUAL GENERAL MEETING OF THE ASSOCIATION OF EUROPEAN PAEDIATRIC CARDIOLOGISTS (AEPC) STOCKHO, XP000891992
- FERRARA N ET AL: "Clinical applications of angiogenic growth factors and their inhibitors." NATURE MEDICINE, (DEC 1999) VOL.5, PP. 1359-64., XP002135826

## Description

### FIELD OF THE INVENTION

The present invention relates to the prevention of stenosis and restenosis of blood vessels, and relates generally to the field of cardiovascular medicine.

### BACKGROUND OF THE INVENTION

Coronary artery disease constitutes a major cause of morbidity and mortality throughout the world, especially in the United States and Europe. Percutaneous transluminal coronary angioplasty (*e.g.*, balloon angioplasty, with our without intracoronary stenting) is now a common and successful therapy for such disease, performed hundreds of thousands of times per year in the United States alone. However, restenosis occurs in as many as one-third to one-half of such revascularization procedures, usually within six months of the angioplasty procedure. The economic cost of restenosis has been estimated at $2 billion annually in the United States alone. [Feldman *et al., Cardiovascular Research, 32:* 194-207 (1996), incorporated herein by reference.] Autopsy and atherectomy studies have identified intimal hyerplasia as the major histologic component of restenotic lesions. [Cerek *et al., Am. J. Cardiol., 68:* 24C-33C (1991).]

Restenosis also remains a clinical concern in angioplasty that is performed in peripheral blood vessels. Likewise, stenosis is a clinical concern following transplantation of blood vessels (e.g., grafted veins and grafted artificial vessels) for cardiac bypass therapy or for treatment of peripheral ischemia or intermittent claudication, for example (e.g., above-knee femoro-popliteal arterial bypass grafts).

Mazur *et al., Texas Heart Institute Journal, 21;* 104-111 (1994) state that restenosis is primarily a response of the artery to the injury caused by percutaneous coronary angioplasty, which disrupts the intimal layer of endothelial cells and underlying smooth muscle cells of the media. The authors state that multiple growth factors secreted by platelets, endothelial cells, macrophages, and smooth muscle cells are mechanistically involved in the restenosis process, and that proliferation of smooth muscle cells constitutes a critical pathogenetic feature. According to the authors, this smooth muscle cell proliferation has proven refractory to mechanical and pharmacologic therapy. More recently, others have called into question whether smooth muscle cell proliferation is of penultimate importance in restenosis. See Libby, *Circ. Res., 82:* 404-406 (1998).

Narins *et al, Circulation, 97:* 1298-1305 (1998) review the use of intracoronary stents and their benefits and limitations in preventing restenosis. Debbas *et al., American Heart Journal, 133*: 460-468 (1997) discuss stenting within a stent to treat in-stent restenosis.

Chang & Leiden, *Semin. Intervent. Cardiol., 1*: 185-193 (1996), incorporated herein by reference, review somatic gene therapy approaches to treat restenosis. Chang and Leiden teach that replication-deficient adenoviruses comprise a promising and safe vector system for gene therapy directed toward prevention of restenosis, because such viruses can efficiently infect a wide variety of cell types, including vascular smooth muscle cells; such viruses can be produced at high titers (*e.g.*, 10¹⁰-10¹² plaque forming units per milliliter); such viruses can accommodate a transgene insert of, *e.g.*, 7-9 kilobases (kb) in size; such viruses can be delivered percutaneously through standard catheters; and such viruses do not integrate into the host genome. Both Chang & Leiden and Feldman *et al., supra,* also review cytotoxic and cytostatic gene therapy approaches, designed to kill or arrest proliferating vascular smooth muscle cells thought to be responsible for neointimal formations that characterize restenosis.

Riessen & Isner, *J. Am. Coll. Cardiol.,* 23:1234-1244 (1994), incorporated by reference, review devices for intravascular drug delivery and vectors for intravascular gene therapy.

Cerek *et al., Am. J. Cardiol., 68:* 24C-33C (1991) suggest prevention of restenosis by inhibiting growth-factor-mediated healing of arterial injury. Potential roles of platelet-derived growth factor (PDGF), thrombospondin, insulin-like growth factor 1 (IGF-1), fibroblast growth factors (FGF's), transforming growth factor alpha (TGF-α) and beta (TGF-β), epidermal growth factor (EGF) are discussed.

Isner & Asahara, International Patent Publication No. WO 98/19712, incorporated herein by reference, suggest treating injured blood vessels and accelerating reendothelialization following angioplasty by isolating a patient's endothelial progenitor cells and re-administering such cells to the patient. The authors suggest that the effectiveness of using an angiogenesis-promoting growth factor, such as vascular endothelial growth factor (VEGF) or basic fibroblast growth factor (bFGF), may be limited by the lack of endothelial cells on which the VEGF or bFGF will exert its effect.

Martin *et al.,* International Patent Publication No. WO 98/20027 suggest the use of VEGF gene or protein to treat or prevent stenosis or restenosis of a blood vessel. The authors suggest that any beneficial effect of VEGF arises from a different mechanism of action than the mechanism underlying an activity of VEGF related to stimulating re-endothelialisation in cases where the endothelium has been damaged.

Callow *et al., Growth Factors, 10:* 223-228 (1994) state that intravenous injection of vascular permeability factor (a.k.a. VEGF) into rabbits that had been subjected to balloon angioplasty-induced endothelial denudation resulted in increased regeneration of endothelium compared to a control. The authors also stated that basic fibroblast growth factor (bFGF) is effective at promoting re-endothelialization, but that such re-endothelialization is accompanied by increases in neointimal lesion size.

Asahara *et al., Circulation, 94*: 3291-3302 (December 15, 1996) state that local, percutaneous catheter delivery of a CMV-human-VEGF₁₆₅ transgene achieved accelerated re-endothelialization in balloon-injured rabbits, and resulted in diminished intimal thickening. In a report by a related group of authors, Van *Belle et al., J. Am. Coll. Cardiol.,* 29:1371-1379 (May, 1997) state that stent endothelialization was accelerated by delivery of a CMV-human-VEGF₁₆₅ transgene and was accompanied by attenuation of intimal thickening.

Morishita *et al., J. Atherosclerosis and Thrombosis, 4(3):* 128-134 (1998) state that hepatocyte growth factor (HGF) has a mitogenic activity on human endothelial cells more potent than VEGF, and hypothesized that HGF gene therapy may have potential therapeutic value for the treatment of cardiovascular diseases such as restenosis after angioplasty. Morishita *et al.* also state that there is little knowledge about growth factors that stimulate only endothelial cells, but not vascular smooth muscle cells.

DeYoung & Dichek, *Circ. Res.,* 82: 306-313 (1998) state that VEGF gene delivery does not currently appear destined for application to human coronary restenosis, and that two independent studies suggest that VEGF delivery may actually worsen arterial intimal hyperplasia.

Brown *et al.,* U.S. Patent No. 5,795,898, suggest using an inhibitor of PDGF, FGF, EGF, or VEGF signaling to suppress accelerated atherogenesis involved in restenosis of coronary vessels or other arterial vessels following angioplasty.

The foregoing discussion demonstrates that a long-felt need continues to exist for improvements to angioplasty materials and/or methods, and/or for adjunct therapies, to reduce instances of restenosis.

### SUMMARY OF THE INVENTION

The present invention addresses long-felt needs in the field of medicine having regard to the prevention of stenosis or restenosis in mammalian blood vessels.

The object of the invention is defined in the claims.

For example, the invention provides the use of a composition in the manufacture of a medicament for treating a mammalian subject to prevent stenosis or restenosis of a blood vessel, said treatment comprising the step of administering to a mammalian subject in need of treatment to prevent stenosis or restenosis of a blood vessel a composition comprising a polynucleotide, the polynucleotide comprising a nucleotide sequence that encodes a vascular endothelial growth factor C (VEGF-C) polypeptide. In a preferred embodiment, the subject is a human subject.

While it is contemplated that the VEGF-C polynucleotide could be administered purely as a prophylactic treatment to prevent stenosis, it is contemplated that the polynucleotide be administered shortly before, and/or concurrently with, and/or shortly after a percutaneous transluminal coronary angioplasty procedure, for the purpose of preventing restenosis of the subject vessel. The polynucleotide can be administered before, during, and/or shortly after a bypass procedure (*e.g.*, a coronary bypass procedure), to prevent stenosis or restenosis in or near the transplanted (grafted) vessel, especially stenosis at the location of the graft itself. The polynucleotide can be administered before, during, or after a vascular transplantation in the vascular periphery that has been performed to treat peripheral ischemia or intermittent claudication. By prevention of stenosis or restenosis is meant prophylactic treatment to reduce the amount/severity of, and/or substantially eliminate, the stenosis or restenosis that frequently occurs in such surgical procedures. The polynucleotide is included in the composition in an amount and in a form effective to promote expression of a VEGF-C polypeptide in a blood vessel of the mammalian subject, thereby preventing stenosis or restenosis of the blood vessel.

In a preferred embodiment, the mammalian subject is a human subject. For example, the subject is a person suffering from coronary artery disease that has been identified by a cardiologist as a candidate who could benefit from a therapeutic balloon angioplasty (with or without insertion of an intravascular stent) procedure or from a coronary bypass procedure. The invention can be used for other mammalian subjects, especially mammals that are conventionally used as models for demonstrating therapeutic efficacy in humans (e.g., primate, porcine, canine, or rabbit animals), also is contemplated.

For the practice of the invention, the term "VEGF-C polypeptide" is intended to include any polypeptide that has a VEGF-C or VEGF-C analog amino acid sequence (as defined elsewhere herein in greater detail) and that possesses in vivo restenosis-reducing effects of human VEGF-C, which effects are demonstrated herein by way of example in a rabbit model. The term "VEGF-C polynucleotide" is intended to include any polynucleotide (*e.g.*, DNA or RNA, single- or double-stranded) comprising a nucleotide sequence that encodes a VEGF-C polypeptide. Due to the well-known degeneracy of the genetic code, there exist multiple VEGF-C polynucleotide sequences that encode any selected VEGF-C polypeptide.

For treatment of humans, VEGF-C polypeptides with an amino acid sequence of a human VEGF-C are highly preferred, and polynucleotides comprising a nucleotide sequence of a human VEGF-C cDNA are highly preferred. By "human VEGF-C" is meant a polypeptide corresponding to a naturally occurring protein (prepro-protein, partially-processed protein, or fully-processed mature protein) encoded by any allele of the human VEGF-C gene, or a polypeptide comprising a biologically active fragment of a naturally-occurring mature protein. By way of example, a human VEGF-C comprises a continuous portion of the amino acid sequence set forth in SEQ ID NO: 2 sufficient to permit the polypeptide to bind and stimulate VEGFR-2 and/or VEGFR-3 phosphorylation in cells that express such receptors. A polypeptide comprising amino acids 131-211 of SEQ ID NO: 2 is specifically contemplated. For example, polypeptides having an amino acid sequence comprising a continuous portion of SEQ ID NO: 2, the continuous portion having, as its amino terminus, an amino acid selected from the group consisting of positions 30-131 of SEQ ID NO: 2, and having, as its carboxyl terminus, an amino acid selected from the group consisting of positions 211-419 of SEQ ID NO: 2 are contemplated. As explained elsewhere herein in greater detail, VEGF-C biological activities, especially those mediated through VEGFR-2, increase upon processing of both an amino-terminal and carboxyl-terminal pro-peptide. Thus, an amino terminus selected from the group consisting of positions 102-131 of SEQ ID NO: 2 is preferred, and an amino terminus selected from the group consisting of positions 103-113 of SEQ ID NO: 2 is highly preferred. Likewise, a carboxyl terminus selected from the group consisting of positions 211-227 of SEQ ID NO: 2 is preferred. As stated above, the term "human VEGF-C" also is intended to encompass polypeptides encoded by allelic variants of the human VEGF-C characterized by the sequences set forth in SEQ ID NOs: 1 & 2.

Moreover, since the therapeutic VEGF-C is to be administered as recombinant VEGF-C or indirectly via somatic gene therapy, it is within the skill in the art to make and use analogs of human VEGF-C (and polynucleotides that encode such analogs) wherein one or more amino acids have been added, deleted, or replaced with other amino acids, especially with conservative replacements, and wherein the anti-restenosis biological activity has been retained. Analogs that retain anti-restenosis VEGF-C biological activity are contemplated as VEGF-C polypeptides for use in the present invention. In a preferred embodiment, analogs having 1, 2, 3, 4, 5, 6, 7, 8, 9, 10, 11, 12, 13, 14, 15, 16, 17, 18, 19, 20, 21, 22, 23, 24, or 25 such modifications and that retain anti-restenosis VEGF-C biological activity are contemplated as VEGF-C polypeptides for use in the present invention. Polynucleotides encoding such analogs are generated using conventional PCR, site-directed mutagenesis, and chemical synthesis techniques.

Also contemplated as VEGF-C polypeptides are non-human mammalian or avian VEGF-C polypeptides and polynucleotides. By "mammalian VEGF-C" is meant a polypeptide corresponding to a naturally occurring protein (prepro-protein, partially-processed protein, or fully-processed mature protein) encoded by any allele of a VEGF-C gene of any mammal, or a polypeptide comprising a biologically active fragment of a mature protein. The term "mammalian VEGF-C polypeptide" is intended to include analogs of mammalian VEGF-C's that possess the *in vivo* restenosis-reducing effects of the mammalian VEGF-C. The fact that gene therapy using a transgene encoding human VEGF-C is effective to prevent restenosis in a rabbit model is evidence of the inter-species therapeutic efficacy of VEGF-C proteins.

Irrespective of which VEGF-C polypeptide is chosen, the VEGF-C polynucleotide preferably comprises a nucleotide sequence encoding a secretory signal peptide fused in-frame with the VEGF-C polypeptide sequence. The secretory signal peptide directs secretion of the VEGF-C polypeptide by the cells that express the polynucleotide, and is cleaved by the cell from the secreted VEGF-C polypeptide. For example, the VEGF-C polynucleotide could encode the complete prepro-VEGF-C sequence set forth in SEQ ID NO: 2; or could encode the VEGF-C signal peptide fused in-frame to a sequence encoding a fully-processed VEGF-C (*e.g.*, amino acids 103-227 of SEQ ID NO: 2) or VEGF-C analog. Moreover, there is no requirement that the signal peptide be derived from VEGF-C. The signal peptide sequence can be that of another secreted protein, or can be a completely synthetic signal sequence effective to direct secretion in cells of the mammalian subject.

In one embodiment, the VEGF-C polynucleotide of the invention comprises a nucleotide sequence that will hybridize to a polynucleotide that is complementary to the human VEGF cDNA sequence specified in SEQ ID NO: 1 under the following exemplary stringent hybridization conditions: hybridization at 42°C in 50% formamide, 5X SSC, 20 mM Na•PO₄, pH 6.8; and washing in 1X SSC at 55°C for 30 minutes; and wherein the nucleotide sequence encodes a polypeptide that binds and stimulates human VEGFR-2 and/or VEGFR-3. It is understood that variation in these exemplary conditions occur based on the length and GC nucleotide content of the sequences to be hybridized. Formulas standard in the art are appropriate for determining appropriate hybridization conditions. *See* Sambrook *et al., Molecular Cloning: A Laboratory Manual* (Second ed., Cold Spring Harbor Laboratory Press, 1989) §§ 9.47-9.51.

In preferred embodiments, the VEGF-C polynucleotide further comprises additional sequences to facilitate the VEGF-C gene therapy. In one embodiment, a "naked" VEGF-C transgene (i.e., a transgene without a viral, liposomal, or other vector to facilitate transfection) is employed for gene therapy. In this embodiment, the VEGF-C polynucleotide preferably comprises a suitable promoter and/or enhancer sequence (*e.g.*, cytomegalovirus promoter/enhancer [Lehner *et al., J. Clin. Microbiol., 29:2494-2502* (1991); Boshart *et al., Cell,* 41:521-530 (1985)]; Rous sarcoma virus promoter [Davis et *al., Hum. Gene Ther.,* 4:151 (1993)]; Tie promoter [Korhonen *et al., Blood, 86(5):* 1828-1835 (1995)]; or simian virus 40 promoter) for expression in the target mammalian cells, the promoter being operatively linked upstream (i.e., 5') of the VEGF-C coding sequence. The VEGF-C polynucleotide also preferably further includes a suitable polyadenylation sequence (e.g., the SV40 or human growth hormone gene polyadenylation sequence) operably linked downstream (i.e., 3') of the VEGF-C coding sequence. The polynucleotide may further optionally comprise sequences whose only intended function is to facilitate large-scale production of the vector, *e.g.*, in bacteria, such as a bacterial origin of replication and a sequence encoding a selectable marker. However, in a preferred embodiment, such extraneous sequences are at least partially cleaved off prior to administration to humans according to methods of the invention. One can manufacture and administer such polynucleotides to achieve successful gene therapy using procedures that have been described in the literature for other transgenes. See, *e.g.,* Isner *et al., Circulation, 91:* 2687-2692 (1995); and Isner *et al., Human Gene Therapy,* 7: 989-1011 (1996); incorporated herein by reference in the entirety.

Any suitable vector may be used to introduce the VEGF-C transgene into the host. Exemplary vectors that have been described in the literature include replication-deficient retroviral vectors, including but not limited to lentivirus vectors [Kim *et al., J. Virol., 72(1):* 811-816 (1998); Kingsman & Johnson, *Scrip Magazine,* October, 1998, pp. 43-46.]; adeno-associated viral vectors [Gnatenko *et al., J. Investig. Med., 45:* 87-98 (1997)]; adenoviral vectors [See, *e.g.,* U.S. Patent No. 5,792,453; Quantin *et al., Proc. Natl. Acad Sci. USA, 89:* 2581-2584 (1992); Stratford-Perricadet *et al., J. Clin. Invest., 90:* 626-630 (1992); and Rosenfeld *et al., Cell, 68:* 143-155 (1992)]; Lipofectin-mediated gene transfer (BRL); liposomal vectors [See, e.g., U.S. Patent No. 5,631,237 (Liposomes comprising Sendai virus proteins)] ; and combinations thereof. All of the foregoing documents are incorporated herein by reference in the entirety. Replication-deficient adenoviral vectors constitute a preferred embodiment.

In embodiments employing a viral vector, preferred polynucleotides still include a suitable promoter and polyadenylation sequence as described above. Moreover, it will be readily apparent that, in these embodiments, the polynucleotide further includes vector polynucleotide sequences (*e.g.*, adenoviral polynucleotide sequences) operably connected to the sequence encoding a VEGF-C polypeptide.

Thus, in one embodiment the composition to be administered comprises a vector, wherein the vector comprises the VEGF-C polynucleotide. In a preferred embodiment, the vector is an adenovirus vector. In a highly preferred embodiment, the adenovirus vector is replication-deficient, i.e., it cannot replicate in the mammalian subject due to deletion of essential viral-replication sequences from the adenoviral genome. For example, the inventors contemplate a method wherein the vector comprises a replication-deficient adenovirus, the adenovirus comprising the VEGF-C polynucleotide operably connected to a promoter and flanked on either end by adenoviral polynucleotide sequences.

The composition to be administered according to the invention preferably comprises (in addition to the polynucleotide or vector) a pharmaceutically-acceptable carrier solution such as water, saline, phosphate-buffered saline, glucose, or other carriers conventionally used to deliver therapeutics intravascularly. Multi-gene therapy is also contemplated, in which case the composition optionally comprises both the VEGF-C polynucleotide/vector and another polynucleotide/vector selected to prevent restenosis. Exemplary candidate genes/vectors for co-transfection with VEGF-C transgenes are described in the literature cited above, including genes encoding cytotoxic factors, cytostatic factors, endothelial growth factors, and smooth muscle cell growth/migration inhibitors. As described in greater detail below, a VEGF-D transgene is a preferred candidate for co-administration with the VEGF-C transgene. Co-administration of a VEGF transgene also is specifically contemplated.

The "administering" that is performed may be performed using any medically-accepted means for introducing a therapeutic directly or indirectly into the vasculature of a mammalian subject, including but not limited to injections; oral ingestion; intranasal or topical administration; and the like. In a preferred embodiment, administration of the composition comprising the VEGF-C polynucleotide is performed intravascularly, such as by intravenous, intra-arterial, or intracoronary arterial injection.

Highly preferable, the composition can be administered locally, *e.g.*, to the site of angioplasty or bypass. For example, the administering comprises a catheter-mediated transfer of the transgene-containing composition into a blood vessel of the mammalian subject, especially into a coronary artery of the mammalian subject. Exemplary materials and methods for local delivery are reviewed in Lincoff *et al., Circulation, 90:* 2070-2084 (1994); and Wilensky *et al., Trends Cardiovasc. Med, 3:163*-170 (1993), both incorporated herein by reference. For example, the composition is administered using infusion-perfusion balloon catheters (preferably mircroporous balloon catheters) such as those that have been described in the literature for intracoronary drug infusions. See, *e.g.*, U.S. Patent No. 5,713,860 (Intravascular Catheter with Infusion Array); U.S. Patent No. 5,087,244; U.S. Patent No. 5,653,689; and Wolinsky *et al., J. Am. Coll. Cardiol., 15:* 475-481 (1990) (Wolinsky Infusion Catheter); and Lambert *et al., Coron. Artery Dis.,* 4: 469-475 (1993). Use of such catheters for site-directed somatic cell gene therapy is described, *e.g.,* in Mazur *et al., Texas Heart Institute Journal, 21;* 104-111 (1994), incorporated herein by reference. In an embodiment where the VEGF-C transgene is to be administered in an adenovirus vector, the vector is preferably administered in a pharmaceutically acceptable carrier at a titer of 10⁷-10¹³ viral particles, and more preferably at a titer of 10⁹- 10¹¹ viral particles. The adenoviral vector composition preferably is infused over a period of 15 seconds to 30 minutes, more preferably 1 to 10 minutes.

For example, in patients with angina pectoris due to a single or multiple lesions in coronary arteries and for whom PTCA is prescribed on the basis of primary coronary angiogram findings, an exemplary protocol involves performing PTCA through a 7F guiding catheter according to standard clinical practice using the femoral approach. If an optimal result is not achieved with PTCA alone, then an endovascular stent also is implanted. (A nonoptimal result is defined as residual stenosis of > 30 % of the luminal diameter according to a visual estimate, and B or C type dissection.) Arterial gene transfer at the site of balloon dilatation is performed with a replication-deficient adenoviral VEGF-C vector immediately after the angioplasty, but before stent implantation, using an infusion-perfusion balloon catheter. The size of the catheter will be selected to match the diameter of the artery as measured from the angiogram, varying, e.g., from 3.0 to 3.5F in diameter. The balloon is inflated to the optimal pressure and gene transfer is performed during a 10 minute infusion at the rate of 0.5 ml/min with virus titer of 1.15 X 10¹⁰.

Intravascular administration with a gel-coated catheter is also contemplated, as has been described in the literature to introduce other transgenes. See, *e.g.*, U.S. Patent No. 5,674,192 (Catheter coated with tenaciously-adhered swellable hydrogel polymer); Riessen *et al., Human Gene Therapy,* 4: 749-758 (1993); and Steg *etal., Circulation, 96:* 408-411 (1997) and *90:* 1648-1656 (1994). Briefly, DNA in solution (*e.g.*, the VEGF-C polynucleotide) is applied one or more times *ex vivo* to the surface of an inflated angioplasty catheter balloon coated with a hydrogel polymer (*e.g.*, Slider with Hydroplus, Mansfield Boston Scientific Corp., Watertown, MA). The Hydroplus coating is a hydrophilic polyacrylic acid polymer that is cross-linked to the balloon to form a high molecular weight hydrogel tightly adhered to the balloon. The DNA covered hydrogel is permitted to dry before deflating the balloon. Re-inflation of the balloon intravascularly, during an angioplasty procedure, causes the transfer of the DNA to the vessel wall.

In yet another embodiment, an expandable elastic membrane or similar structure mounted to or integral with a balloon angioplasty catheter or stent is to be employed to deliver the VEGF-C transgene. See, e.g., U.S. Patent Nos. 5,707,385, 5,697,967, 5,700,286, 5,800,507, and 5,776,184.

In another variation, the composition containing the VEGF-C transgene is to be administered extravascularly, e.g., using a device to surround or encapsulate a portion of vessel. See, e.g., International Patent Publication WO 98/20027 describing a collar that is placed around the outside of an artery (*e.g.*, during a bypass procedure) to deliver a transgene to the arterial wall via a plasmid or liposome vector.

Endothelial cells or endothelial progenitor cells can also be transfected *ex vivo* with the VEGF-C transgene, and the transfected cells are administered to the mammalian subject. Exemplary procedures for seeding a vascular graft with genetically modified endothelial cells are described in U.S. Patent No. 5,785,965.

If the mammalian subject is receiving a vascular graft, the VEGF-C transgene-containing composition may be directly applied to the isolated vessel segment prior to its being grafted *in vivo*.

In another aspect, the invention provides the use of a composition in the manufacture of a medicament for treating a mammalian subject to prevent stenosis or restenosis of a blood vessel, said treatment comprising the step of administering to a mammalian subject in need of treatment to prevent stenosis or restenosis of a blood vessel a composition comprising a VEGF-C polypeptide, in an amount effective to prevent stenosis or restenosis of the blood vessel. In a preferred embodiment, the administering comprises implanting an intravascular stent in the mammalian subject, where the stent is coated or impregnated with the composition. Exemplary materials for constructing a drug-coated or drug-impregnated stent are described in literature cited above and reviewed in Lincoff *et al., Circulation, 90:* 2070-2084 (1994). In another preferred embodiment, the composition comprises microparticles composed of biodegradable polymers such as PGLA, non-degradable polymers, or biological polymers (e.g., starch) which particles encapsulate or are impregnated by the VEGF-C polypeptide. Such particles are delivered to the intravascular wall using, e.g., an infusion angioplasty catheter. Other techniques for achieving locally sustained drug delivery are reviewed in Wilensky *et al., Trends Caridovasc. Med., 3:*163-170 (1993), incorporated herein by reference.

Administration via one or more intravenous injections subsequent to the angioplasty or bypass procedure also is contemplated. Localization of the VEGF-C polypeptides to the site of the procedure occurs due to expression of VEGF-C receptors on proliferating endothelial cells. Localization is further facilitated by recombinantly expressing the VEGF-C as a fusion polypeptide (e.g., fused to an apolipoprotein B-100 oligopeptide as described in Shih *et al., Proc. Nat'l. Acad. Sci. USA, 87:1436-1440* (1990). Co-administration of VEGF-C polynucleotides and VEGF-C polypeptides also is contemplated.

In yet another embodiment, the invention provides the use of a VEGF-C polynucleotide or VEGF-C polypeptide for the manufacture of a medicament for the treatment or prevention of stenosis or restenosis of a blood vessel.

In still another embodiment, the invention provides the use of a composition in the manufacture of a medicament for treating a mammalian subject to prevent stenosis or restenosis of a blood vessel, said treatment comprising the step of administering to a mammalian subject in need of treatment to prevent stenosis or restenosis of a blood vessel a composition comprising a polynucleotide, the polynucleotide comprising a nucleotide sequence that encodes a vascular endothelial growth factor D (VEGF-D) polypeptide. Such methods are practiced essentially as described herein with respect to VEGF-C-encoding polynucleotides, except that polynucleotides encoding VEGF-D are employed. A detailed description of the human VEGF-D gene and protein are provided in Achen, *et al., Proc. Nat'l Acad. Sci. U.S.A., 95(2):* 548-553 (1998); International Patent Publication No. WO 98/07832, published 26 February 1998; and in Genbank Accession No. AJ000185. A cDNA and deduced amino acid sequence for prepro-VEGF-D is set forth herein in SEQ ID NOs: 3 and 4. Of course, due to the well-known degeneracy of the genetic code, there exist multiple VEGF-D encoding polynucleotide sequences, any of which may be employed according to the methods taught herein.

As described herein in detail with respect to VEGF-C, the use of polynucleotides that encode VEGF-D fragments, VEGF-D analogs, VEGF-D allelic and interspecies variants, and the like which possess *in vivo* anti-restenosis effects of human VEGF-D are all contemplated as being encompassed by the present invention.

In yet another embodiment, the invention provides the use of a composition in the manufacturing of a medicament for treating a mammalian subject to prevent stenosis or restenosis of a blood vessel, said treatment comprising the step of administering to a mammalian subject in need of treatment to prevent stenosis or restenosis of a blood vessel a composition comprising a VEGF-D polypeptide, in an amount effective to prevent stenosis or restenosis of the blood vessel. Such methods are practiced essentially as described herein with respect to VEGF-C polypeptides.

In a related aspect, the invention provides materials and devices for practice of the above-described methods.

Likewise, the invention also provides surgical devices that are used to treat circulatory disorders, such as intravascular (endovascular) stents, balloon catheters, infusion-perfusion catheters, extravascular collars, elastomeric membranes, and the like, which have been improved by coating with, impregnating with, adhering to, or encapsulating within the device a composition comprising a VEGF-C polynucleotide, a VEGF-C polypeptide, a VEGF-D polynucleotide, and/or a VEGF-D polypeptide.

For example, in one embodiment, the invention provides an endovascular stent characterized by an improvement wherein the stent is coated or impregnated with a composition, the comprising at least one anti-restenosis agent selected from the group consisting of VEGF-C polynucleotides, VEGF-C polypeptides, VEGF-D polynucleotides, and VEGF-D polypeptides. Exemplary stents that may be improved in this manner are described and depicted in U.S. Patent Nos. 5,800,507 and 5,697,967 (Medtronic, Inc., describing an intraluminal stent comprising fibrin and an elutable drug capable of providing a treatment of restenosis); U. S. Patent No. 5,776,184 (Medtronic, Inc., describing a stent with a porous coating comprising a polymer and a therapeutic substance in a solid or solid/solution with the polymer); U.S. Patent No. 5,799,384 (Medtronic, Inc., describing a flexible, cylindrical, metal stent having a biocompatible polymeric surface to contact a body lumen); U.S. Patent Nos. 5,824,048 and 5,679,400; and U.S. Patent No. 5,779,729. Implantation of such stents during conventional angioplasty techniques will result in less restenosis than implantation of conventional stents. In this sense, the biocompatibility of the stent is improved.

In another embodiment, the invention provides an extravascular collar for delivery of a therapeutic agent to a blood vessel, characterized by an improvement wherein the collar is coated with or impregnated with or encapsulates a composition, the comprising at least one anti-restenosis agent selected from the group consisting of VEGF-C polynucleotides, VEGF-C polypeptides, VEGF-D polynucleotides, and VEGF-D polypeptides. An exemplary collar to be improved in this manner is described and depicted in International Patent Publication WO 98/20027 (Eurogene, Ltd., collar comprising a body adopted to provide a seal around a vessel and to define a reservoir for holding an anti-restenosis pharmaceutical formulation).

In yet another embodiment, the invention provides a polymer film for wrapping a stent, characterized by an improvement wherein the film is coated with or impregnated with a composition, the comprising at least one anti-restenosis agent selected from the group consisting of VEGF-C polynucleotides, VEGF-C polypeptides, VEGF-D polynucleotides, and VEGF-D polypeptides. An exemplary film to be improved in this manner is described and depicted in U.S. Patent Nos. 5,700,286 and 5,707,385 (Advanced Cardiovascular Systems, Inc., sheaths of bioabsorbable polymeric material coated or impregnated with a restenosis-preventing therapeutic agent and attachable to an endovascular stent).

Similarly, the invention includes kits which comprise compounds or compositions of the invention packaged in a manner which facilitates their use to practice methods of the invention. In a simplest embodiment, such a kit includes a compound or composition described herein as useful for practice of the invention (e.g., VEGF-C or VEGF-D polynucleotides or polypeptides), packaged in a container such as a sealed bottle or vessel, with a label affixed to the container or included in the package that describes use of the compound or composition to practice the method of the invention. Preferably, the compound or composition is packaged in a unit dosage form. In another embodiment, a kit of the invention includes both a VEGF-C or VEGF-D polynucleotide or polypeptide composition packaged together with a physical device useful for implementing methods of the invention, such as a stent, a catheter, an extravascular collar, a polymer film, or the like. In another embodiment, a kit of the invention includes both a VEGF-C or VEGF-D polynucleotide or polypeptide composition packaged together with a hydrogel polymer, or microparticle polymers, or other carriers described herein as useful for delivery of the VEGF-C/VEGF-D to the patient.

Additional features and variations of the invention will be apparent to those skilled in the art from the entirety of this application, and all such features are intended as aspects of the invention.

Likewise, features of the invention described herein can be re-combined into additional embodiments that also are intended as aspects of the invention, irrespective of whether the combination of features is specifically mentioned above as an aspect or embodiment of the invention. Also, only such limitations which are described herein as critical to the invention should be viewed as such; variations of the invention lacking limitations which have not been described herein as critical are intended as aspects of the invention.

### BRIEF DESCRIPTION OF THE DRAWING

Figure 1 depicts a cross-section of a blood vessel into which a drug delivery balloon catheter including a protective sheath has been inserted, the protective sheath serving to cover the balloon during insertion and positioning.
Figure 2A depicts a perspective view of an expandable membrane having two layers that are spaced apart, prior to joining edges of the layers to each other.
Figure 2B depicts a perspective view of the membrane of Figure 2A that has been rolled into a tube and had opposite edges adjoined.
Figures 3A and 3B depict, in perspective (3A) and longitudinal cross-section (3B), schematic views of an extravascular collar surrounding a portion of a blood vessel.
Figure 4A depicts in cross-section a wire coated with a polymer or gel that can include (e.g., be impregnated with) a therapeutic composition.
Figure 4B depicts a perspective view of an intravascular stent formed from the wire of Figure 4A.

### DETAILED DESCRIPTION OF THE INVENTION

The present invention is based on the discovery that when a gene encoding human Vascular Endothelial Growth Factor C (VEGF-C) is administered to a mammal that has suffered a vascular trauma, such as the trauma that can occur during conventional balloon angioplasty procedures, restenosis of the injured vessel is reduced or eliminated. An *in vivo* controlled experiment demonstrating the efficacy of a VEGF-C transgene to prevent restenosis is described in detail in Example 1. Example 2 provides a side-by-side comparative study demonstrating that the anti-restenosis effects of VEGF-C appear superior to the anti-restenosis effects of VEGF administered in a comparable manner.

The growth factor named Vascular Endothelial Growth Factor C (VEGF-C), as well as native human, non-human mammalian, and avian polynucleotide sequences encoding VEGF-C, and VEGF-C variants and analogs, have been described in detail in International Patent Application Number PCT/US98/01973, filed 02 February 1998 and published on 06 August 1998 as International Publication Number WO 98/33917; in Joukov *et al., J. Biol. Chem., 273(12):* 6599-6602 (1998); and in Joukov *et al., EMBO J., 16(13):* 3898-3911 (1997). As explained therein in detail, human VEGF-C is initially produced in human cells as a prepro-VEGF-C polypeptide of 419 amino acids. A cDNA and deduced amino acid sequence for human prepro-VEGF-C are set forth in SEQ ID NOs: 1 and 2, respectively, and a cDNA encoding human VEGF-C has been deposited with the American Type Culture Collection (ATCC), 10801 University Blvd., Manassas, VA 20110-2209 (USA), pursuant to the provisions of the Budapest Treaty (Deposit date of 24 July 1995 and ATCC Accession Number 97231). VEGF-C sequences from other species also have been reported. See Genbank Accession Nos. MMU73620 (Mus musculus); and CCY15837 (Coturnix coturnix) for example.

The prepro-VEGF-C polypeptide is processed in multiple stages to produce a mature and most active VEGF-C polypeptide of about 21-23 kD (as assessed by SDS-PAGE under reducing conditions). Such processing includes cleavage of a signal peptide (SEQ ID NO: 2, residues 1-31); cleavage of a carboxyl-terminal peptide (corresponding approximately to amino acids 228-419 of SEQ ID NO: 2 and having a pattern of spaced cysteine residues reminiscent of a Balbiani ring 3 protein (BR3P) sequence [Dignam *et al., Gene, 88*:133-40 (1990); Paulsson *et al., J. Mol. Biol., 211*:331-49 (1990)]) to produce a partially-processed form of about 29 kD; and cleavage (apparently extracellularly) of an amino-terminal peptide (corresponding approximately to amino acids 32-103 of SEQ ID NO: 2) to produced a fully-processed mature form of about 21-23 kD. Experimental evidence demonstrates that partially-processed forms of VEGF-C (*e.g.*, the 29 kD form) are able to bind the Flt4 (VEGFR-3) receptor, whereas high affinity binding to VEGFR-2 occurs only with the fully processed forms of VEGF-C. It appears that VEGF-C polypeptides naturally associate as non-disulfide linked dimers.

Moreover, it has been demonstrated that amino acids 103-227 of SEQ ID NO: 2 are not all critical for maintaining VEGF-C functions. A polypeptide consisting of amino acids 113-213 (and lacking residues 103-112 and 214-227) of SEQ ID NO: 2 retains the ability to bind and stimulate VEGF-C receptors, and it is expected that a polypeptide spanning from about residue 131 to about residue 211 will retain VEGF-C biological activity. The cysteine residue at position 156 has been shown to be important for VEGFR-2 binding ability. However, VEGF-C ΔC₁₅₆ polypeptides (i.e., analogs that lack this cysteine due to deletion or substitution) remain potent activators of VEGFR-3. If the anti-restenosis effects of VEGF-C are mediated through VEGFR-3, then use of VEGF-C ΔC₁₅₆ polypeptides (and polynucleotides encoding them) is expected to provide anti-restenosis efficacy while minimizing VEGFR-2-mediated side-effects. The cysteine at position 165 of SEQ ID NO: 2 is essential for binding either receptor, whereas analogs lacking the cysteines at positions 83 or 137 compete with native VEGF-C for binding with both receptors and stimulate both receptors.

An alignment of human VEGF-C with VEGF-C from other species (performed using any generally accepted alignment algorithm) suggests additional residues wherein modifications can be introduced (*e.g.*, insertions, substitutions, and/or deletions) without destroying VEGF-C biological activity. Any position at which aligned VEGF-C polypeptides of two or more species have different amino acids, especially different amino acids with side chains of different chemical character, is a likely position susceptible to modification without concomitant elimination of function. An exemplary alignment of human, murine, and quail VEGF-C is set forth in Figure 5 of PCT/US98/01973.

Apart from the foregoing considerations, it will be understood that innumerable conservative amino acid substitutions can be performed to a wildtype VEGF-C sequence which are likely to result in a polypeptide that retains VEGF-C biological activities, especially if the number of such substitutions is small. By "conservative amino acid substitution" is meant substitution of an amino acid with an amino acid having a side chain of a similar chemical character. Similar amino acids for making conservative substitutions include those having an acidic side chain (glutamic acid, aspartic acid); a basic side chain (arginine, lysine, histidine); a polar amide side chain (glutamine, asparagine); a hydrophobic, aliphatic side chain (leucine, isoleucine, valine, alanine, glycine); an aromatic side chain (phenylalanine, tryptophan, tyrosine); a small side chain (glycine, alanine, serine, threonine, methionine); or an aliphatic hydroxyl side chain (serine, threonine). Addition or deletion of one or a few internal amino acids without destroying VEGF-C biological activities also is contemplated.

Without intending to be limited to a particular theory, the mechanism behind the efficacy of VEGF-C in preventing restenosis is believed to relate to the ability of VEGF-C to stimulate re-endothelialization of the injured vessel (and/or of the intravascular stent) without significant concomitant stimulation of smooth muscle proliferation in the vessel. VEGF-C also may inhibit smooth muscle cell proliferation. Accordingly, candidate VEGF-C analog polypeptides can be rapidly screened first for their ability to bind and stimulate autophosphorylation of known VEGF-C receptors (VEGFR-2 and VEGFR-3). Polypeptides that stimulate one or both known receptors are rapidly rescreened *in vitro* for their mitogenic and/or chemotactic activity against cultured capillary or arterial endothelial cells (*e.g.*, as described in WO 98/33917). Polypeptides with mitogenic and/or chemotactic activity are then screened *in vivo* as described herein for the ability to prevent restenosis. In this way, variants (analogs) of naturally occurring VEGF-C proteins are rapidly screened to determine whether or not the variants have the requisite biological activity to constitute "VEGF-C polypeptides" for use in the present invention.

The growth factor named Vascular Endothelial Growth Factor D (VEGF-D), as well as human sequences encoding VEGF-D, and VEGF-D variants and analogs, have been described in detail in International Patent Application Number PCT/US97/14696, filed 21 August 1997 and published on 26 February 1998 as International Publication Number WO 98/07832; and in Achen, *et al., Proc. Nat'l Acad Sci. U.S.A., 95(2):* 548-553 (1998), both incorporated herein by reference in the entirety. As explained therein in detail, human VEGF-D is initially produced in human cells as a prepro-VEGF-D polypeptide of 354 amino acids. A cDNA and deduced amino acid sequence for human prepro-VEGF-D are set forth in SEQ ID NOs: 3 and 4, respectively. VEGF-D sequences from other species also have been reported. See Genbank Accession Nos. D89628 (Mus musculus); and AF014827 (Rattus norvegicus), for example, incorporated herein by reference.

The prepro-VEGF-D polypeptide has a putative signal peptide of 21 amino acids and is apparently proteolytically processed in a manner analogous to the processing of prepro-VEGF-C. A "recombinantly matured" VEGF-D lacking residues 1-92 and 202-354 of SEQ ID NO: 4 retains the ability to activate receptors VEGFR-2 and VEGFR-3, and appears to associate as non-covalently linked dimers. Thus, preferred VEGF-D polynucleotides include those polynucleotides that comprise a nucleotide sequence encoding amino acids 93-201 of SEQ ID NO: 4. The guidance provided above for introducing function-preserving modifications into VEGF-C polypeptides is also suitable for introducing function-preserving modifications into VEGF-D polypeptides.

A therapeutic or prophylactic treatment of restenosis involves administering to a mammalian subjection such as a human a composition comprising a VEGF-C or VEGF-D polynucleotide or polypeptide or combination thereof (sometimes generically referred to herein as a "VEGF-C or VEGF-D therapeutic agent").

The "administering" may be performed using any medically-accepted means for introducing a therapeutic directly or indirectly into the vasculature of a mammalian subject, including but not limited to injections; oral ingestion; intranasal or topical administration; and the like. In a preferred embodiment, administration of the composition comprising the VEGF-C or VEGF-D polynucleotide or polypeptide composition is performed intravascularly, such as by intravenous, intra-arterial, or intracoronary arterial injection.

Highly preferably, the composition may be administered locally, e.g., to the site of angioplasty or bypass. For example, the administering comprises a catheter-mediated transfer of the therapeutic composition into a blood vessel of the mammalian subject, especially into a coronary artery of the mammalian subject. Exemplary materials and methods for local delivery are reviewed in Lincoff *et al., Circulation, 90:* 2070-2084 (1994); and Wilensky *et al., Trends Cardiovasc. Med, 3*:163-170 (1993). For example, the composition is administered using infusion-perfusion balloon catheters (preferably mircroporous balloon catheters) such as those that have been described in the literature for intracoronary drug infusions. See, e.g., U.S. Patent No. 5,713,860 (Intravascular Catheter with Infusion Array); U.S. Patent No. 5,087,244; U.S. Patent No. 5,653,689; and *Wolinsky et al., Am. Coll. Cardiol., 15:* 475-481 (1990) (Wolinsky Infusion Catheter); and Lambert *et al., Coron. Artery Dis.,* 4: 469-475 (1993). Use of such catheters for site-directed somatic cell gene therapy is described, e.g., in Mazur *et al., Texas Heart Institute Journal, 21;* 104-111 (1994).

For example, in patients with angina pectoris due to a single or multiple lesions in coronary arteries and for whom PTCA is prescribed on the basis of primary coronary angiogram findings, an exemplary protocol involves performing PTCA through a 7F guiding catheter according to standard clinical practice using the femoral approach. If an optimal result is not achieved with PTCA alone, then an endovascular stent also is implanted. (A nonoptimal result is defined as residual stenosis of > 30 % of the luminal diameter according to a visual estimate, and B or C type dissection.) Arterial gene transfer at the site of balloon dilatation is performed immediately after the angioplasty, but before stent implantation, using an infusion-perfusion balloon catheter. The size of the catheter will be selected to match the diameter of the artery as measured from the angiogram, varying, *e.g.*, from 3.0 to 3.5F in diameter. The balloon is inflated to the optimal pressure and gene transfer is performed during a 10 minute infusion at the rate of 0.5 ml/min with virus titer of 1.15 X 10¹⁰.

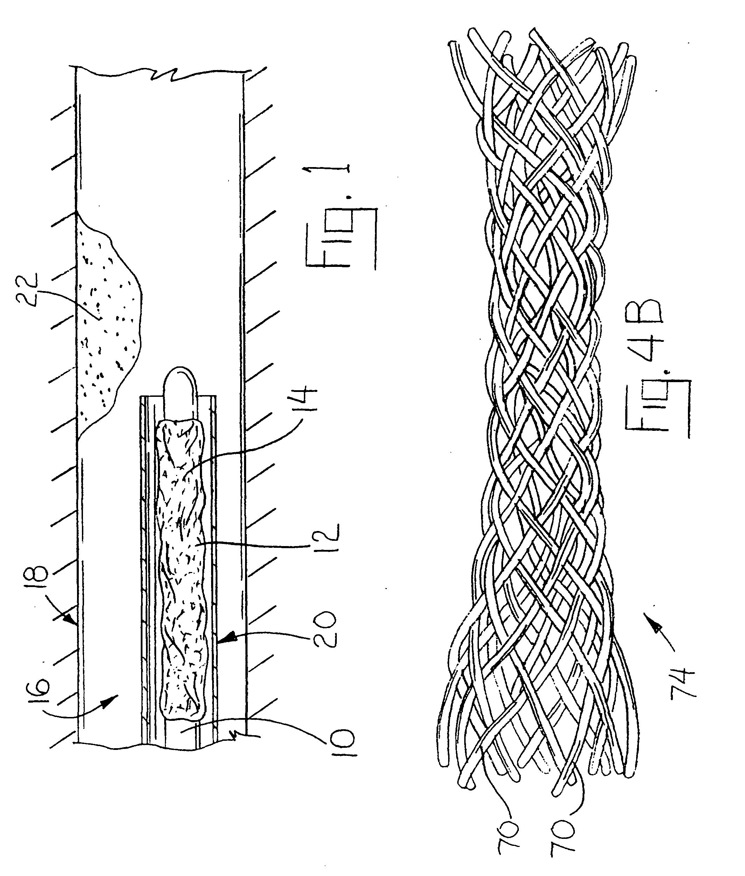

Intravascular administration with a gel-coated catheter is also contemplated, as has been described in the literature to introduce other transgenes. See, *e.g.*, U.S. Patent No. 5,674,192 (Catheter coated with tenaciously-adhered swellable hydrogel polymer); Riessen *et al., Human Gene Therapy, 4*: 749-758 (1993); and Steg *et al., Circulation, 96:* 408-411 (1997) and *90:* 1648-1656 (1994); all incorporated herein by reference. As shown in Figure 1, a catheter **10** is provided to which an inflatable baloon **12** is attached at a distal end. The balloon includes a swellable hydrogel polymer coating **14** capable of absorbing a solution comprising a therapeutic VEGF-C or VEGF-D therapeutic agent. Briefly, DNA in solution (*e.g.*, the VEGF-C or VEGF-D polynucleotide) is applied one or more times *ex vivo* to the surface of an inflated angioplasty catheter balloon coated with a hydrogel polymer (e.g., Slider with Hydroplus, Mansfield Boston Scientific Corp., Watertown, MA). The Hydroplus coating is a hydrophilic polyacrylic acid polymer that is cross-linked to the balloon to form a high molecular weight hydrogel tightly adhered to the balloon. The DNA covered hydrogel is permitted to dry before deflating the balloon. Re-inflation of the balloon intravascularly, during an angioplasty procedure, causes the transfer of the DNA to the vessel wall. Thus, referring again to Figure 1, the catheter with attached, coated balloon is inserted into the lumen **16** of a blood vessel **18** while covered by a protective sheath **20** to minimize exposure of the coated balloon to the blood prior to placement at the site of an occlusion **22**. When the instrument has been positioned at the treatment region, the protective sheath is drawn back or the catheter is moved forward to expose the balloon, which is inflated to compress the balloon (and thus the coating) into the vessel wall, causing transfer of the VEGF-C or VEGF-D therapeutic agent to the tissue, in a manner analogous to squeezing liquid from a compressed sponge or transferring wet paint to a surface by contact.

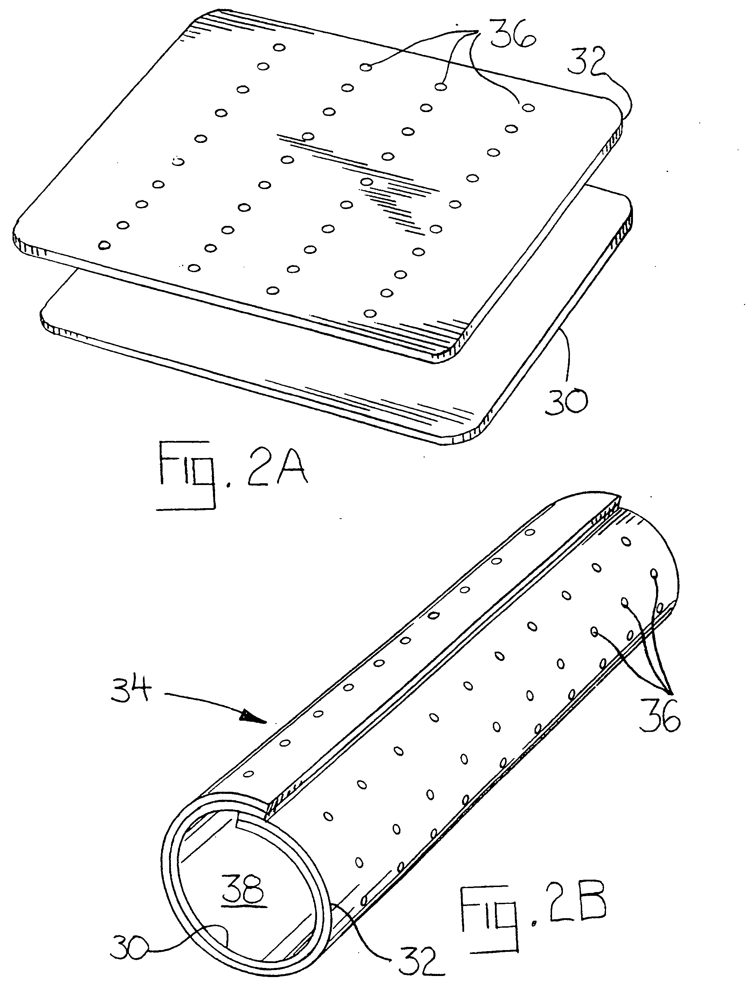

In yet another embodiment, an expandable elastic membrane, film, or similar structure, mounted to or integral with a balloon angioplasty catheter or stent, is to be employed to deliver the VEGF-C or VEGF-D therapeutic agent. See, *e.g.*, U.S. Patent Nos. 5,707,385, 5,697,967, 5,700,286, 5,800,507, and 5,776,184. As shown in Figures 2A-2B, a single layer **30** or multi-layer **30, 32** sheet of elastic membrane material (Fig. 2A) is formed into a tubular structure **34** (Fig. 2B), *e.g.,* by bringing together and adhering opposite edges of the sheet(s), e.g., in an overlapping or a abutting relationship. In this manner the elastomeric material may be wrapped around a catheter balloon or stent. A therapeutic VEGF-C or VEGF-D composition is combined with the membrane using any suitable means, including injection molding, coating, diffusion, and absorption techniques. In the multilayer embodiment depicted in the Figures, the edges of the two layers may be joined to form a fluid-tight seal. In a preferred embodiment, one layer of material is first processed by stretching the material and introducing a plurality of microscopic holes or slits **36**. After the layers have been joined together, the sheet can be stretched and injected with the therapeutic VEGF-C/D composition through one of the holes or slits to fill the cavity that exists between the layers. The sheet is then relaxed, causing the holes to close and sealing the therapeutic composition between the layers until such time as the sheet is again stretched. This occurs, for example, at the time that an endovascular stent or balloon covered by the sheet is expanded within the lumen of a stenosed blood vessel. The expanding stent or balloon presses radially outward against the inner surface **38** of the tubular sheet covering, thus stretching the sheet, opening the holes, and delivering the therapeutic agent to the walls of the vessel.

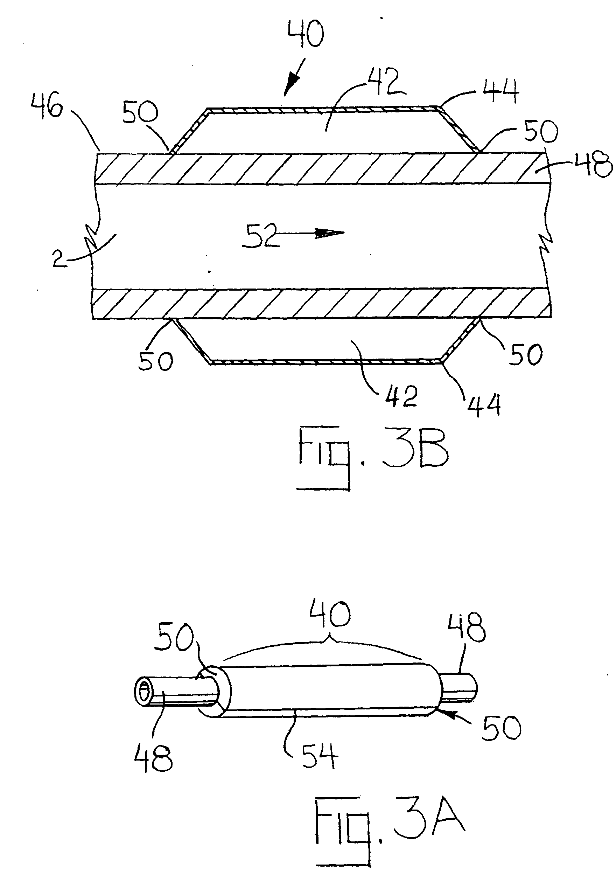

In another variation, the composition containing the VEGF-C or VEGF-D therapeutic is to be administered extravascularly, e.g., using a device to surround or encapsulate a portion of vessel. See, e.g., International Patent Publication WO 98/20027, incorporated herein by reference, describing a collar that is placed around the outside of an artery (*e.g.*, during a bypass procedure) to deliver a transgene to the arterial wall via a plasmid or liposome vector. As shown in Figures 3A and 3B, an extravascular collar **40** including a void space **42** defined by a wall **44** formed, e.g., of a biodegradable or biocompatible material. The collar touches the outer wall **46** of a blood vessel **48** at the collar's outer extremities **50**. Blood **52** flows through the lumen of the blood vessel. A longitudinal slit **54** in the flexible collar permits the collar to be deformed and placed around the vessel and then sealed using a conventional tissue glue, such as a thrombin glue.

In still another variation, endothelial cells or endothelial progenitor cells can be transfected *ex vivo* with the VEGF-C a VEGF-D transgene, and the transfected cells as administered to the mammalian subject. Exemplary procedures for seeding a vascular graft with genetically modified endothelial cells are described in U.S. Patent No. 5,785,965.

If the mammalian subject is receiving a vascular graft, the VEGF-C or VEGF-D therapeutic composition may be directly applied to the isolated vessel segment prior to its being grafted *in vivo.*

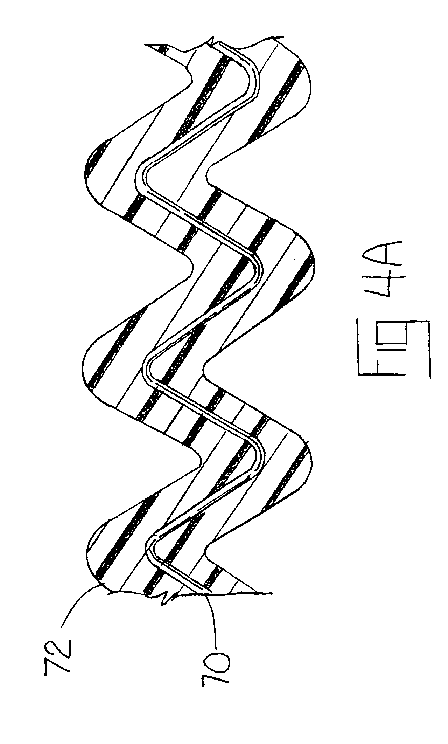

In another preferred embodiment, an intravascular stent is to be implanted in the mammalian subject, where the stent is coated or impregnated with the therapeutic VEGF-C/D gene/protein composition. Exemplary materials for constructing a drug-coated or drug-impregnated stent are described in literature cited above and reviewed in Lincoff *et al., Circulation, 90:* 2070-2084 (1994). As shown in Figure 4A and 4B, a metal or polymeric wire **70** for forming a stent is coated with a composition **72** such as a porous biocompatible polymer or gel that is impregnated with (or can be dipped in or otherwise easily coated immediately prior to use with) a VEGF-C or VEGF-D therapeutic composition. The wire is coiled, woven, or otherwise formed into a stent **74** suitable for implanation into the lumen of a vessel using conventional materials and techniques, such as intravascular angioplasty catheterization. Exemplary stents that may be improved in this manner are described and depicted in U.S. Patent Nos. 5,800,507 and 5,697,967 (Medtronic, Inc., describing an intraluminal stent comprising fibrin and an elutable drug capable of providing a treatment of restenosis); U.S. Patent No. 5,776,184 (Medtronic, Inc., describing a stent with a porous coating comprising a polymer and a therapeutic substance in a solid or solid/solution with the polymer); U.S. Patent No. 5,799,384 (Medtronic, Inc., describing a flexible, cylindrical, metal stent having a biocompatible polymeric surface to contact a body lumen); U.S. Patent Nos. 5,824,048 and 5,679,400; and U.S. Patent No. 5,779,729. Implantation of such stents during conventional angioplasty techniques will result in less restenosis than implantation of conventional stents. In this sense, the biocompatibility of the stent is improved.

In another preferred embodiment, the composition comprises microparticles composed of biodegradable polymers such as PGLA, non-degradable polymers, or biological polymers (e.g., starch) which particles encapsulate or are impregnated by the VEGF-C or VEGF-C polypeptide/polynucleotide. Such particles are delivered to the intravascular wall using, e.g., an infusion angioplasty catheter. Other techniques for achieving locally sustained drug delivery are reviewed in Wilensky *et al., Trends Caridovasc. Med.,* 3:163-170 (1993).

Administration via one or more intravenous injections subsequent to the angioplasty or bypass procedure also is contemplated. Localization of the VEGF-C or VEGF-D polypeptides to the site of the procedure occurs due to expression of VEGF-C/D receptors on proliferating endothelial cells. Localization is further facilitated by recombinantly expressing the VEGF-C or VEGF-D as a fusion polypeptide (*e.g.*, fused to an apolipoprotein B-100 oligopeptide as described in Shih *et al., Proc. Nat'l. Acad Sci. USA,* 87:1436-1440 (1990).

The pharmaceutical efficacy of VEGF-C polynucleotides, VEGF-C polypeptides, VEGF-D polynucleotides, and VEGF-D polypeptides to prevent stenosis or restenosis of a blood vessel is demonstrated *in vivo, e.g.,* using procedures such as those described in the following examples, some of which are prophetic. The examples assist in further describing the invention, but are not intended in any way to limit the scope of the invention.

### Example 1

### Use of adenovirus-mediated VEGF-C gene transfer to prevent restenosis

The following experiments, performed *in vivo* in a rabbit restenosis model, demonstrate the efficacy of adenovirus-mediated intravascular VEGF-C gene transfer for the prevention of post-angioplasty restenosis.

### A. Materials and Methods

### 1. Adenoviral constructs.

An adenovirus plasmid containing a cDNA encoding the complete human prepro-VEGF-C open reading frame operably linked to a cytomegalovirus (CMV) promoter and human growth hormone polyadenylation signal sequence was constructed as follows. A DNA fragment comprising a CMV promoter sequence was prepared by digesting the pcDNA3.1+ vector (Invitrogen) with *Sal* and filling-in the 5' overhangs with the Klenow enzyme. The CMV promoter (nucleotides 5431-911) was excised from the vector with *Hind* III and isolated. A full-length human VEGF-C cDNA containing the 1997 bp sequence specified in SEQ ID NO: 1 (as well as less than 50 bases of additional non-coding and polylinker sequence) was excised from a VEGF-C pREP7 expression vector [described in WO 98/33917] with *Hind* III and *Xho* I and isolated. A human growth hormone polyadenylation signal (~ 860 bp) was excised from an αMHC vector with *Sal*I and *Bam*HI*.* The CMV promoter, VEGF-C cDNA, and hGH polyadenylation signal fragments were simultaneously ligated into a *BamHI* and *Eco*RV-digested pCRII vector. The ligated CMV promoter and VEGF-C cDNA is shown in SEQ ID NO: 17. The resulting construct was opened with *Bgl*II and partially-digested with *BamHI.* The full transcriptional unit was ligated into *Bgl*II-opened pAdBglII vector. This construct [designated pAdBg1II VEGF-C] was then used to create recombinant adenovirus containing the CMV-VEGF-C-hGH transcriptional unit, using standard homologous recombination techniques. *[Barr et al., Gene Ther.,* 1: 51-58 (1994).] Replication-deficient E1-E3 deleted adenoviruses were produced in 293 cells and concentrated by ultracentrifugation using techniques known in the literature. [See, *e.g.,* Barr *et al.* (1994).] A control plasmid comprising the *lacz* gene operably linked to the same promoter was also used. [Laitinen M. *et al., Hum. Gene Ther., 9:* 1481-1486 (1998).] The *lacZ* adenovirus had a nuclear targeted signal, to direct the β-galactosidase expression to the nucleus. Replication-deficient E1-E3 deleted adenoviruses were produced in 293 cells and concentrated by ultracentrifugation (Barr *et al.,* 1994). The adenoviral preparations were analyzed for the absence of helper viruses and bacteriological contaminants.

### 2. Animal model.

New Zealand White rabbits were employed for the gene transfer study. A first group of rabbits was fed a 0.25 % cholesterol diet for two weeks, then subjected to balloon denudation of the aorta, then subjected three days later to the adenovirus-mediated gene transfer. A second group of rabbits was only subjected to the gene transfer. Animals were sacrificed 2 or 4 weeks after the gene transfer. The number of experimental (VEGF-C) and control (*lacZ*) animals in both study groups was 6.

In the first group of rabbits, the whole aorta, beginning from the tip of the arch, was denuded using a 4.0 F arterial embolectomy catheter (Sorin Biomedical, Irvine, CA). The catheter was introduced via the right iliac artery up to the aortic arch and inflated, and the aorta was denuded twice.

### 3. Gene transfer.

The gene transfer was performed using a 3.0 F channel balloon local drug delivery catheter (Boston Scientific Corp., Maple Grove, MA). Using fluoroscopical control, the balloon catheter was positioned caudal to the left renal artery, in a segment free of side branches, via a 5 F percutaneous introducer sheath (Arrow International, Reading, PA) in the right carotid artery and inflated to 6 ATM with a mixture of contrast media and saline. The anatomical location of the balloon catheter was determined by measuring its distance from the aortic orifice of the left renal artery. Virus titer of 1.15 x 10¹⁰ plaque forming units (pfu) was administered to each animal in a final volume of 2 ml (0.9 % NaCl), and the gene transfer was performed at 6 ATM pressure for 10 minutes (0.2 ml / min). In the second study group the animals had only gene transfer and they were sacrificed 2 weeks after the gene transfer. The number of animals in each study group (0.9 % NaCl only; *lacZ* gene transfer; and VEGF-C gene transfer) was 3. All studies were approved by Experimental Animal Committee of the University of Kuopio in Finland.

### 4. Histology.

Three hours before sacrifice, the animals were injected intravenously with 50 mg ofBrdU dissolved in 40 % ethanol. After the sacrifice, the aortic segment where the gene transfer had been performed was removed, flushed gently with saline, and divided into five equal segments. The proximal segment was snap frozen in liquid nitrogen and stored at -70°C. The next segment was immersion-fixed in 4 % paraformaldehyde /15 % sucrose (pH 7.4) for 4 hours, rinsed in 15 % sucrose (pH 7.4) overnight, and embedded in paraffin. The medial segment was immersion-fixed in 4% paraformaldehyde / phosphate buffered saline (PBS) (pH 7.4) for 10 minutes, rinsed 2 hours in PBS, embedded in OCT compound (Miles), and stored at -70°C. The fourth segment was immersion-fixed in 70 % ethanol overnight and embedded in paraffin. The distal segment was directly stained for β-galactosidase activity in X-GAL staining solution at +37°C for 16 hours, immersion-fixed in 4 % paraformaldehyde /15 % sucrose (pH 7.4) for 4 hours, rinsed in 15 % sucrose overnight, and embedded in paraffin. Paraffin sections were used for immunocytochemical detection of smooth muscle cells (SMC), macrophages, and endothelium. Gene transfer efficiency was evaluated using X-GAL staining ofOCT-embedded tissues. BrdU-positive cells were detected according to manufacturer's instructions. Morphometry was performed using haematoxylin-eosin stained paraffin sections using image analysis software. Measurements were taken independently by two observers from multiple sections, without knowledge of the origin of the sections. Intima/media (I/M) ratio was used as a parameter for intimal thickening.

### B. Results.

Histological analysis of the balloon-denuded mice revealed that the *lacZ*-transfected control group had an I/M ratio of 0.61 two weeks after the gene transfer, which represented a statistically significant difference (p< 0.05) from the VEGF-C-transfected groups (I/M ratio of 0.40). The tendency that VEGF-C group had a smaller I/M ratio persisted at 4 weeks time point after the gene transfer.

In the second group of rabbits that were subjected only to gene transfer to the vessel wall (without endothelial denudation), the I/M ratio in the *lacZ* group was 0.3, compared to 0.15 for the VEGF-C group. This difference, too, represented a statistically significant (p< 0.05) inhibition in neointima formation in VEGF-C group.

The BrdU labeling will permit analysis of smooth muscle cell proliferation in VEGF-C-transfected versus control *(lacZ)* animals. SMC proliferation is expected to be reduced in the VEGF-C-transfected population.

The foregoing data demonstrate that VEGF-C gene transfer significantly reduced intimal thickening at two weeks time point after aortic denudation and after vessel wall damage caused by the gene transfer catheter without balloon denudation. These data indicate a therapeutic utility for VEGF-C gene transfer for the prevention of post-angioplasty restenosis.

### Example 2

### Comparative Example Demonstrating that Anti-Restenosis Effects of VEFG-C Appear Superior to Those of VEGF.

The following experiments demonstrate the efficacy of adenovirus-mediated intravascular VEGF and VEGF-C gene transfer for the prevention of post-angioplasty restenosis, and demonstrates that VEGF-C appeared to provide a superior therapeutic efficacy compared to VEGF.

### A. Materials and Methods

### 1. Adenoviral constructs.

VEGF (murine VEGF-A₁₆₄; SEQ ID NO: 18) adenovirus was constructed using the same promoter as the VEGF-C construct, and following similar procedure as described in Example 1. The VEGF-A₁₆₄ adenoviral construct was produced in 293T cells and concentrated essentially as described in Example 1, and analyzed to be free of helper virus, lipopolysaccharides, and bacterial contaminants.

### 2. Animal model.

Sixty three New Zealand White rabbits were divided into two major groups, the first having 0.25% cholesterol diet for two weeks and balloon denudation of the aorta before gene transfer, and the second group having only the gene transfer. Gene transfer was performed in the first group of rabbits three days after denudation, and the animals were sacrificed 2 or 4 weeks after the gene transfer. Number of rabbits in each study group *(lacZ,* VEGF, and VEGF-C) at both time points was 6. In the second study group, the rabbits had only the gene transfer, without cholesterol diet or balloon denudation, and were sacrificed 2 or 4 weeks after the gene transfer. The number of rabbits in each study group (0.9% saline, *lacZ,* VEGF, and VEGF-C) was 3.

### 3. Gene transfer.

Gene transfer was performed according to the procedure described in Example 1.

### 4. Histology

Histology was performed essentially as described in Example 1 with the following modifications: SMC were detected using HHF35 (DAKO, 1:50 dilution), macrophages were detected using RAM-11 (DAKO, 1:50 dilution), endothelium was detected using CD31 (DAKO, 1:50 dilution), and T cells were detected using MCA 805 (DAKO, 1:100 dilution). Controls for immunostainings included incubations with class-and species matched immunoglobulins and incubations where primary antibodies were omitted. Morphometry and image analysis were performed using Image-Pro Plus™ software and an Olympus AX70 microscope (Olympus Optical, Japan). Statistical analyses were performed using the ANOVA and modified t-test. P<0.05 was considered statistically significant.

### B. Results

Histological analysis of the balloon-denuded rabbit aorta shows intimal thickening and SMC proliferation. Two weeks after gene transfer, the *lacZ* control group had the highest I/M ratio (0.57 ± 0.04) whereas VEGF-C (0.38 ± 0.02) and VEGF (0.49± 0.17) groups showed decreased intimal thickening. The difference in I/M ratios between *lacZ* and VEGF-C groups was significant (*P*<0.05), whereas those between *lacZ* and VEGF groups were not statistically significant, at the two-week time point. The tendency that both VEGF and VEGF-C groups had smaller I/M ratios persisted at the four week time point when the I/M ratio was 0.73 ± 0.16, 0.44 ± 0.14, and 0.63 ± 0.21 for the lacZ, VEGF-C, and VEGF groups, respectively. Hematoxylin-eosin and immunostainings of the transfected arteries indicate that intimal thickening in all arteries was composed predominantly of SMC.

Use of adenoviral vectors can lead to immnological and inflammatory responses, partly because high titer adenovirus induces expression of NFκB and activates a CTL response. However, no signs of inflammation nor foam cell accumulation were detected as judged by macrophage and T-cell immunostainings. In addition, human clinical gene therapy grade viruses were used together with short exposure times in the transfected arteries, which may also help explain the absence of severe inflammatory reactions in this study.

The percentage of proliferating cells was analyzed using BrdU labeling. No significant differences were seen, although the VEGF-C group tended to have a lower proliferation rate, consistent with the observation that VEGF-C transduced arteries had smaller I/M ratios at both time points. Two weeks after balloon denudation, the percentage of proliferating cells was 1.8 ± 0.4, 2.2 ± 0.7, and 1.2 ± 0.0 for the *lacz,* VEGF, and VEGF-C groups, respectively, and after four weeks, the percentage of proliferating cells was 0.3 ± 0.1, 1.2 ± 0.5, and 0.3 ± 0.1 for the *lacZ,* VEGF, and VEGF-C groups, respectively. Endothelial regrowth was analyzed by measuring the length of intact endothelium from histological sections. No significant differences were found between the study groups.

The potential of adenovirus to cause damage to the vessel wall and neointima formation was tested by performing high-titer adenovirus gene transfer to intact abdominal aorta of rabbits without balloon-denudation. Control rabbits were treated in the same way with 0.9% saline. The positioning of the gene transfer catheter caused some internal elastic lamina damage and moderate induction of neoinitma formation after the procedure. At the two-week time point, the I/M ratio in the *lacZ* group was 0.24 ± 0.06, in the control group 0.28 ± 0.05, in the VEGF-C group 0.18 ± 0.07, and in the VEGF group 0.15 ± 0.03. At the four-week time point the *lacZ* group had an I/M ratio of 0.22 ± 0.13, the VEGF-C group 0.13 ± 0.03, and the VEGF group 0.23 ± 0.11.

This study shows a beneficial therapeutic effect of intravascular adenovirus-mediated VEGF-C gene transfer on the vessel wall after balloon injury, and also compares VEGF-C and VEGF adenovirus-mediated gene transfer for the prevention of neointima formation. Although different receptor binding profiles of VEGF-C and VEGF might have led to different biological effects in the vessel wall, both VEGFs reduced intimal thickening two weeks after gene transfer. Thus, both VEGFs are potential candidates for vascular gene therapy of ischemic atherosclerotic diseases. However, according to this experiment, VEGF-C appears to prevent restenosis more effectively than VEGF in this model system. The superior ability of VEGF-C to prevent restenosis, as compared to VEGF, could be due to expression or activity of VEGFR-3 which is a receptor for VEGF-C and VEGF-D, but not for VEGF. Alternatively, the apparent superiority may be attributable to a restenosis-promoting effect of VEGF mediated through VEGFR-1 or due to differential ligand effects (VEGF-C versus VEGF) mediated thru the common receptor VEGFR-2, which is reportedly expressed in vascular smooth muscle cells. [See Grosskreutz *et al., Microvasc. Res., 58(2):* 128-136 (September, 1999).

### Example 3

### Expression of transfected VEGFs in the aortic wall

Using the aortic segments from the same experimental animals described in Example 2, mRNA expression of *lacZ,* VEGF-C and VEGF (murine VEGF-A₁₆₄) was analyzed in aortic tissue after gene transfer. Total RNA was extracted from transfected aortic segments using Trizol Reagent (Gibco-BRL), and 2 µg of RNA was used for cDNA synthesis. Primers for *lacZ,* VEGF-C and VEGF were designed to distinguish between endogenous and transduced genes by selecting the 5' primers from the CMV promoter and the 3' primers from the coding regions.

For *lacZ* amplification, primers were: 5' primer 5'-TTGGAGGCCTAGGCTTTTGC-3' (SEQ ID NO: 5) and 3' primer 5'-ATACTGTCGTCGTCCCCTCA-3' (SEQ ID NO: 6). The first PCR cycle was an initial incubation at 96°C for 4 minutes followed by 80°C for 3 minutes during which the DNA polymerase was added. This was followed by 30 cycles, each consisting of 94°C for 45 seconds, 58°C for 45 seconds, and 72°C for 50 seconds, followed by a final extension of 72°C for 5 minutes. 5 µl of the first PCR product was used for the second PCR with 5' primer 5'-GGTAGAAGACCCCAAGGACTTT-3'(SEQ ID NO: 7) and 3' primer 5'-CGCCATTCGCCATTCAG-3' (SEQ ID NO: 8). The first PCR cycle was an initial incubation at 96°C for 3 minutes followed by 80°C for 3 minutes followed by 32 cycles, each consisting of 94°C for 60 seconds, 58°C for 15 seconds, and 72°C for 90 seconds, followed by a final extension of 72°C for 5 minutes.

For VEGF-C amplification, primers were: 5' primer 5'-CTGCTTACTGGCTTATCG-3' (SEQ ID NO: 9) and 3' primer 5'-CCTGTTCTCTGTTATGTTGC-3' (SEQ ID NO: 10). The first PCR cycle was an initial incubation at 96°C for 4 minutes followed by 80°C for 3 minutes during which the DNA polymerase was added. This was followed by 39 cycles each consisting of 94°C for 30 seconds, 56°C for 40 seconds, and 72°C for 90 seconds, followed by a final extension of 72°C for 5 minutes. 5 µl of the first PCR product was used for the second PCR with 5' primer 5'-TCTCCAAAAAGCTACACCG-3' (SEQ ID NO: 11) and 3' primer 5'-CAAGTGCATGGTGGAAGG-3' (SEQ ID NO: 12). The first PCR cycle was an initial incubation at 96°C for 3 minutes followed by 80°C for 3 minutes followed by 39 cycles each consisting of 94°C for 60 seconds, 57°C for 30 seconds, and 72°C for 90 seconds, followed by a final extension of 72°C for 5 minutes.

For VEGF amplification, primers were: 5' primer 5'-TCGATCCATGAACTTTCTGC-3' (SEQ ID NO: 13) and 3' primer 5'-TTCGTTTAACTCAAGCTGCC-3' (SEQ ID NO: 14). The first PCR cycle was an initial incubation at 96°C for 4 minutes followed by 80°C for 3 minutes, followed by 39 cycles each consisting of 94°C for 30 seconds, 53°C for 40 seconds, and 72°C for 90 seconds, followed by a final extension of 72°C for 5 minutes. 5 µl of the first PCR product was used for the second PCR with 5' primer 5'-GACCCTGGCTTTACTGCTG-3' (SEQ ID NO: 15) and 3' primer 5'-GGAACATTTACACGTCTGCG-3' (SEQ ID NO: 16). The first PCR cycle was an initial incubation at 96°C for 3 minutes followed by 80°C for 3 minutes followed by 39 cycles each consisting of 94°C for 60 seconds, 54°C for 30 seconds, and 72°C for 90 seconds, followed by a final extension of 72°C for 5 minutes.

The mRNA of *lacZ,* VEGF-C and VEGF was detected in aortic wall tissue up to four weeks after gene transfer.

Gene transfer efficiency was evaluated by assaying *lacZ* expression, analyzed by X-Gal staining for β-galactosidase activity, in OCT embedded tissue sections. Transfection efficiency was 1.1% ± 0.5 and 0.3% ± 0.1, two and four weeks respectively, after intravascular catheter-mediated gene transfer.

### Example 4

### Expression of VEGF receptors in the aortic wall

Using the experimental animals described in Example 2, VEGFR-1, VEGFR-2, and VEGFR-3 expression in aortic tissue was analyzed by immunostainings and *in situ* hybridization. Immunohisochemistry was performed using clone sc-316 (Santa Cruz Biotechnology, 1:50 dilution) to detect VEGFR-1, clone sc-6251 (Santa Cruz Biotechnology, 1:500 dilution) to detect VEGFR-2, and clone sc-637 (Santa Cruz Biotechnology, 1:300 dilution) to detect VEGFR-3. Controls for immunostainings included incubations with class- and species matched immunoglobulins and incubations where primary antibodies were omitted. *In situ* hybridization of VEGF receptor mRNAs was carried out using ³³P-UTP labeled riboprobes. Expression of all receptors was localized to endothelium. VEGFR-2 was also expressed in neointimal SMCs.

### Example 5

### Use of naked VEGF-C transgene therapy to prevent restenosis

The procedures described in Example 1 or 2 are repeated, with the following modifications. Instead of using an adenovirus vector for delivery of the VEGF-C transgene, a mammalian expression vector is constructed for direct gene transfer (of naked plasmid DNA). The VEGF-C coding sequence is operably linked to a suitable promoter, such as the CMV promoter, and preferably linked to a suitable polyadenylation sequence, such as the human growth hormone polyadenylation sequence. Exemplary VEGF-C vectors can be modeled from vectors that have been described in the literature to perform vector-free gene transfer for other growth factors, by substituting a VEGF-C coding sequence for a VEGF coding sequence. [See, *e.g.,* Isner *et al., Circulation, 91:* 2687-2692 (1995); and Isner *et al., Human Gene Therapy,* 7: 989-1011 (1996), incorporated herein by reference.] vector. A similar construct comprising a *lacz* gene is used as a control.

A Hydrogel-coated balloon catheter (Boston Scientific) is used to deliver the VEGF-C transgene essentially as described in Asahara *et al., Circulation,* 94: 3291-3302 (December 15, 1996), incorporated herein by reference. Briefly, an angioplasty balloon is prepared *ex vivo* by advancing the deflated balloon completely through a teflon protective sheath (Boston Scientific). The balloon is inflated and a conventional pipette is used to apply the transgene construct (*e.g.*, 50 - 5000 µg transgene DNA in a saline solution) to the Hydrogel polymer coating the external surface of the inflated balloon. After the transgene solution has dried, the balloon is deflated, withdrawn into the protective sheath, and re-inflated to minimize blood flow across the balloon surface until the balloon is properly positioned in the target artery.

Intima/media (I/M) ratio is again used as a parameter for intimal thickening. Reduced I/M ratio in animals treated with the VEGF-C transgene-coated balloon catheter is considered indicative of therapeutic efficacy. As described in Example 2, comparison of the therapeutic efficacy of VEGF-C gene transfer with other therapies, such as VEGF gene transfer, can be conducted in parallel.

### Example 6

### Use ofVEGF-C gene therapy to prevent restenosis following angioplasty with stent

The procedures described in the preceding examples are repeated with the modification that initial balloon angioplasty is accompanied by implantation of a coronary stent using conventional procedures. The VEGF-C transgene is delivered concurrently or immediately before or after stent implantation essentially as described in the preceding examples. Increased quantities (*e.g.*, two-fold to ten-fold) of the transgene (compared to angioplasty without stent) and increased transfection time may be desirable, as described in Van Belle *et al., J. Am. Coll. Cardiol.,* 29:1371-1379 (May, 1997), incorporated by reference herein. Decreased neointimal thickening and/or decreased thrombotic occlusion in the VEGF-C gene-treated animals versus control animals treated with a marker gene is considered evidence of the efficacy of the VEGF-C gene therapy.

### Example 7

### Use of an extravascular collar to reduce vascular stenosis.

An inert silicone collar such as described in International Patent Publication No. WO 98/20027 is surgically implanted around the carotid arteries of New Zealand White Rabbits. The collar acts as an irritation agent that will induce intimal thickening, and contains a reservoir suitable for local delivery of a VEGF-C transgene or protein pharmaceutical formulation. Gene transfer, using the VEGF-C adenovirus construct or control construct described in Example 1 is initiated five days later by injecting 10⁸- 10¹¹ pfu into the collar. Animals are sacrificed 14 or 28 days later and histological examinations are performed as described in Example 1. Intima/media thickness ratio [Yla-Herttuala *et al., Arteriosclerosis,* 6: 230-236 (1986)] is used as an indicia of stenosis. Reduced I/M ratio in the VEGF-C-transfected rabbits, as compared to the *lacZ* control rabbits, indicates therapeutic efficacy of VEGF-C gene transfer for preventing arterial stenosis.

### Example 8

### Use of VEGF-C polypeptides to reduce or prevent restenosis

The procedures described in Example 1 are repeated except, instead of treating the test animals with an adenovirus containing a VEGF-C transgene or *lacZ* control, the animals are treated with a composition comprising a VEGF-C polypeptide in a pharmaceutically acceptable carrier (*e.g.*, isotonic saline with serum albumim), or with carrier solution alone as a control. Test animals receive either 10, 100, 250, 500, 1000, or 5000 µg of a VEGF-C polypeptide via intra-arterial infusion, *e.g.*, as described in Example 1. A second group of animals additionally receive an injection of the VEGF-C polypeptide 7 days later. The animals are sacrificed and histological examination performed as described in Example 1. Reduced I/M ratio in the VEGF-C-treated animals versus control animals provides evidence of the therapeutic efficacy of VEGF-C polypeptide treatment. Repetition of the experiment using various sustained-release VEGF-C formulations and materials as described above is expected to further enhance the therapeutic efficacy of the VEGF-C polypeptide. Moreover, a treatment regimen comprising the simultaneous administration of VEGF-C protein (to provide immediate therapy to the target vessel) with a VEGF-C transgene (to provide sustained therapy for several days or weeks) is specifically contemplated as a variation of the invention.

### Example 9

### Anti-stenosis/anti-restenosis activity of VEGF-D

The procedures described in the preceding examples are repeated using a composition comprising a VEGF-D polynucleotide or VEGF-D polypeptide in lieu of the VEGF-C polynucleotide/polypeptide, to demonstrate the ability of VEGF-D to prevent stenosis or restenosis of a blood vessel.

While the present invention has been described in terms of specific embodiments, it is understood that variations and modifications will occur to those in the art, all of which are intended as aspects of the present invention. Accordingly, only such limitations as appear in the claims should be placed on the invention.

## Claims

1. Use of a composition in the manufacture of a medicament for treating or preventing restenosis of a blood vessel in a mammalian subject, preferably a human, wherein the composition comprises at least one agent selected from the group consisting of: polynucleotides comprising a nucleotide sequence that encodes a vascular endothelial growth factor C (VEGF-C) polypeptide; polynucleotides comprising a nucleotide sequence that encodes a vascular endothelial growth factor D (VEGF-D) polypeptide; VEGF-C polypeptide; and VEGF-D polypeptide.

2. A use according to claim 1, wherein said agent comprises a polynucleotide that comprises a nucleotide sequence encoding a mammalian VEGF-C polypeptide, preferably a human VEGF-C polypeptide, or a nucleotide sequence encoding a mammalian VEGF-D polypeptide, preferably a human VEGF-D polypeptide.

3. A use according to claim 2, wherein when the nucleotide sequence encodes a human VEGF-C polypeptide comprising an amino acid sequence including a continuous portion of SEQ ID NO: 2, said continuous portion having, as its amino terminus, an amino acid selected from the group consisting of positions 30-131 of SEQ ID NO: 2, and having, as its carboxyl terminus, an amino acid selected from the group consisting of positions 211 to 419 of SEQ ID NO: 2.

4. A use according to claim 3, wherein said polynucleotide lacks a nucleotide sequence encoding amino acids 228-419 and/or amino acids 32-102 of SEQ ID NO:2.

5. A use according to claim 3 or 4, wherein said polynucleotide further comprises a nucleotide sequence encoding a secretory signal peptide, wherein the sequence encoding the secretory signal peptide is connected in-frame with the sequence that encodes the VEGF-C polypeptide.

6. A use according to claim 5, wherein the polynucleotide further comprises a promoter sequence operably connected to the sequence that encodes the secretory signal sequence and VEGF-C polypeptide, wherein the promoter sequence promotes transcription of the sequence that encodes the secretory signal sequence and the VEGF-C polypeptide in mammalian cells.

7. A use according to claim 6, wherein the polynucleotide further comprises a polyadenylation sequence operably connected to the sequence that encodes the VEGF-C polypeptide.

8. A use according to any one of claims 1-7, wherein the composition comprises a gene therapy vector, said gene therapy vector including said polynucleotide, said vector optionally comprising a replication-deficient adenovirus, said adenovirus comprising the polynucleotide operably connected to a promoter and flanked by adenoviral polynucleotide sequences.

9. A use according to any one of claims 1-8, wherein the composition further comprises a pharmaceutically acceptable carrier.

10. A use according to any one of claims 1-9, wherein the medicament is formulated for administration:-
(a) in at least one intravascular injection; or
(b) in a catheter-mediated transfer of said composition into a blood vessel of the mammalian subject, preferably through the introduction of a catheter into a coronary artery of the mammalian subject, and release the composition into the contrary artery.

11. A use according to any one of claims 1-10, wherein the medicament is formulated for concurrent administration, to said human, with a percutaneous transluminal coronary angioplasty.

12. A use according to claim 10 or 11, wherein the medicament is coated onto or impregnated in an intravascular stent.

13. Use of a composition in the manufacture of a medicament for preventing restenosis of a blood vessel in a human, wherein the composition comprises a replication-deficient adenovirus vector that includes a polynucleotide encoding at least one polypeptide selected from the group consisting of VEGF-C polypeptides, and VEGF-D polypeptides, and further comprising a promoter sequence to promote expression of the at least one polypeptide in cells of the blood vessel.

14. A use according to claim 15, wherein the polynucleotide encode a VEGF-C polypeptide.

15. An endovascular stent designed to contact a surface of a blood vessel in the course of surgery to treat stenosis of the blood vessel, the stent comprising an outer surface for contacting a surface of a blood vessel, and a composition on said surface comprising a composition as claimed in any one of claims 1-15.

16. An extravascular collar designed to contact a surface of a blood vessel in the course of surgery to treat stenosis of the blood vessel, the collar comprising an outer wall shaped to surround the outer surface of a blood vessel, wherein the wall encloses a space containing a composition as claimed in any one of claims 1-15.

17. A kit for treating restenosis comprising a container holding a composition as defined in claim 1 or claim 14, a label attached to or packaged with the container, the label describing use of the composition for prevention of restenosis of a blood vessel, and:-
(a) a medical device selected from the group consisting of: intravascular stents, intravascular catheters, extravascular collars, and membranes adapted to cover a surface of an intravascular stent or catheter; and/or
(b) a carrier substance for delivery of the agent to the lumenal wall of a vessel, preferably wherein the carrier is selected from the group consisting of hydrogel polymers and microparticle polymers.

## Patentansprüche

1. Verwendung einer Zusammensetzung zur Herstellung eines Arzneimittels zur Behandlung oder Verhinderung von Restenose eines Blutgefäßes bei einem Säugetierpatienten, vorzugsweise einem Menschen, wobei die Zusammensetzung mindestens ein Mittel umfaßt, das ausgewählt ist aus der Gruppe, bestehend aus: Polynukleotiden, die eine Nukleotidsequenz umfassen, die für das Polypeptid eines vaskulären endothelialen Machstumsfaktors C (VEGF-C) kodiert; Polynukleotiden, die eine Nukleotidsequenz umfassen, die für das Polypeptid eines vaskulären endothelialen Wachstumsfaktors D (VEGF-D) kodiert; VEGF-C-Polypeptid; und VEGF-D-Polypeptid.

2. Verwendung nach Anspruch 1, wobei das Mittel ein Polynukleotid umfaßt, das eine Nukleotidsequenz umfaßt, die ein Säugetier-VEGF-C-Polypeptid, bevorzugt ein menschliches VEGF-C-Polypeptid, kodiert, oder eine Nukleotidsequenz, die ein Säugetier-VEGF-D-Polypeptid, bevorzugt ein menschliches VEGF-D-Polypeptid, kodiert.

3. Verwendung nach Anspruch 1, wobei, wenn die Nukleotidsequenz für ein menschliches VEGF-C-Polypeptid kodiert, das eine Aminosäuresequenz umfaßt, die einen zusammenhängenden Bereich der SEQ ID NO: 2 enthält, der zusammenhängende Bereich an seinem Aminoterminus eine Aminosäure aufweist, die ausgewählt ist aus der Gruppe, bestehend aus den Positionen 30-131 der SEQ ID NO: 2 und an seinem Carboxyterminus eine Aminosäure aufweist, die ausgewählt ist aus der Gruppe, bestehend aus den Positionen 211 bis 419 der SEQ ID NO: 2.

4. Verwendung nach Anspruch 3, wobei dem Polynukleotid eine Nukleotidsequenz fehlt, die für die Aminosäuren 228-419 und/oder die Aminosäuren 32-102 der SEQ ID NO: 2 kodiert.

5. Verwendung nach einem der Ansprüche 3 oder 4, wobei das Polynukleotid ferner eine Nukleotidsequenz umfaßt, die für ein sekretorisches Signalpeptid kodiert, wobei die Sequenz, die für das sekretorische Signalpeptid kodiert, mit der Sequenz, die für das VEGF-C-Polypeptid kodiert, "in-frame" verbunden ist.

6. Verwendung nach Anspruch 5, wobei das Polynukleotid ferner eine Promotorsequenz umfaßt, die mit der Sequenz, die für die sekretorische Signalsequenz und das VEGF-C-Polypeptid kodiert, funktionsfähig verbunden ist, wobei die Promotorsequenz die Transkription der Sequenz, die für die sekretorische Signalsequenz und das VEGF-C-Polypeptid kodiert, in Säugetierzellen fördert.

7. Verwendung nach Anspruch 6, wobei das Polynukleotid ferner eine Poly-A-Sequenz umfaßt, die mit der Sequenz, die für das VEGF-C-Polypeptid kodiert, funktionsfähig verbunden ist.

8. Verwendung nach einem der Ansprüche 1-7, wobei die Zusammensetzung einen Gentherapie-Vektor umfaßt, wobei der Gentherapie-Vektor das Polynukleotid enthält, und wobei der Vektor optional einen replikationsdefizienten Adenovirus umfaßt, wobei der Adenovirus das Polynukleotid funktionsfähig mit einem Promotor verbunden und durch eine adenovirale Polynukleotidsequenz flankiert umfaßt.

9. Verwendung nach einem der Ansprüche 1-8, wobei die Zusammensetzung ferner einen pharmazeutisch annehmbaren Träger umfaßt.

10. Verwendung nach einem der Ansprüche 1-9, wobei das Arzneimittel formuliert ist zur Verabreichung:
(a) in mindestens einer intravaskulären Injektion; oder
(b) in einem kathetervermittelten Transfer der Zusammensetzung in ein Blutgefäß eines Säugetierpatienten, bevorzugt durch Einführen eines Katheters in eine Koronararterie des Säugetierpatienten und Freisetzen der Zusammensetzung in die Koronararterie.

11. Verwendung nach einem der Ansprüche 1-10, wobei das Arzneimittel formuliert ist für die gleichzeitige Verabreichung mit einer perkutanen transluminalen Koronarangioplastie.

12. Verwendung nach Anspruch 10 oder 11, wobei ein intravaskulärer Stent mit dem Arzneimittel beschichtet oder imprägniert wird.

13. Verwendung einer Zusammensetzung zur Herstellung eines Arzneimittels zur Verhinderung von Restenose eines Blutgefäßes bei einem Menschen, wobei die Zusammensetzung einen replikationsdefizienten Adenovirus-Vektor umfaßt, der ein Polynukleotid enthält, das mindestens ein Polypeptid kodiert, das ausgewählt ist aus der Gruppe, bestehend aus VEGF-C-Polypeptiden und VEGF-D-Polypeptiden, und ferner eine Promotorsequenz zur Förderung der Expression von dem mindestens einen Polypeptid in Zellen des Blutgefäßes umfaßt.

14. Verwendung nach Anspruch 15, wobei das Polynukleotid ein VEGF-C-Polypeptid kodiert.

15. Endovaskulärer Stent, angepaßt zur Kontaktierung einer Oberfläche eines Blutgefäßes im Verlauf einer Operation zur Behandlung der Stenose eines Blutgefäßes, wobei der Stent eine äußere Oberfläche zur Kontaktierung einer Oberfläche eines Blutgefäßes und eine Zusammensetzung auf der Oberfläche umfaßt, umfassend eine Zusammensetzung nach einem der Ansprüche 1-15.

16. Extravaskuläre Manschette, angepaßt zur Kontaktierung einer Oberfläche eines Blutgefäßes im Verlauf einer Operation zur Behandlung der Stenose eines Blutgefäßes, wobei die Manschette eine äußere Wand umfaßt, die so gestaltet ist, daß sie die äußere Oberfläche eines Blutgefäßes umgibt, wobei die Wand einen Raum einschließt, der eine Zusammensetzung nach einem der Ansprüche 1-15 enthält.

17. Kit zur Behandlung von Restenose, umfassend einen Behälter, der eine wie in Anspruch 1 oder Anspruch 14 definierte Zusammensetzung enthält, ein an dem Behälter angebrachtes oder damit verpacktes Etikett, wobei das Etikett die Verwendung der Zusammensetzung zur Verhinderung von Restenose eines Blutgefäßes beschreibt, und:
(a) eine medizinische Vorrichtung, ausgewählt aus der Gruppe, bestehend aus: intravaskulären Stents, intravaskulären Kathetern, extravaskulären Manschetten und Membranen, die angepaßt sind, eine Oberfläche eines intravaskulären Stents oder Katheters zu bedecken; und/oder
(b) eine Trägersubstanz zur Zuführung des Mittels an die Lumenwand eines Gefäßes, wobei der Träger bevorzugt ausgewählt ist aus der Gruppe, bestehend aus Hydrogelpolymeren und Mikropartikelpolymeren.

## Revendications

1. Utilisation d'une composition dans la production d'un médicament destiné au traitement ou à la prévention d'une resténose d'un vaisseau sanguin chez un sujet consistant en un mammifère, de préférence l'homme, dans laquelle la composition comprend au moins un agent choisi dans le groupe consistant en : des polynucléotides comprenant une séquence de nucléotides qui code pour un polypeptide facteur de croissance endothéliale vasculaire C (VEGF-C) ; des polynucléotides comprenant une séquence de nucléotides qui code pour un polypeptide facteur de croissance endothéliale vasculaire D (VEGF-D) ; le polypeptide VEGF-C et le polypeptide VEGF-D.

2. Utilisation suivant la revendication 1, dans laquelle ledit agent comprend un polynucléotide qui comprend une séquence de nucléotides codant pour un polypeptide VEGF-C de mammifère, de préférence un polypeptide VEGF-C humain, ou une séquence de nucléotides codant pour un polypeptide VEGF-D de mammifère, de préférence un polypeptide VEGF-D humain.

3. Utilisation suivant la revendication 2, dans laquelle, lorsque la séquence de nucléotides code pour un polypeptide VEGF-C humain comprenant une séquence d'aminoacides comprenant une partie continue de la SEQ ID N° 2, ladite partie continue ayant, à son extrémité amino, un aminoacide choisi dans le groupe consistant en les aminoacides aux positions 30-131 de la SEQ ID N° 2, et ayant, à son extrémité carboxyle, un aminoacide choisi dans le groupe consistant en les aminoacides aux positions 211 à 419 de la SEQ ID N° 2.

4. Utilisation suivant la revendication 3, dans laquelle ledit polynucléotide est dépourvu d'une séquence de nucléotides codant pour les aminoacides 228-419 et/ou les aminoacides 32-102 de la SEQ ID N° 2.

5. Utilisation suivant la revendication 3 ou 4, dans laquelle ledit polynucléotide comprend en outre une séquence de nucléotides codant pour un peptide signal de sécrétion, la séquence codant pour le peptide signal de sécrétion étant connectée en cadre avec la séquence qui code pour le polypeptide VEGF-C.

6. Utilisation suivant la revendication 5, dans laquelle le polynucléotide comprend en outre une séquence de promoteur connectée de manière fonctionnelle à la séquence qui code pour la séquence signal de sécrétion et le polypeptide VEGF-C, la séquence de promoteur servant de promoteur de transcription de la séquence qui code pour la séquence signal de sécrétion et le polypeptide VEGF-C dans des cellules de mammifère.

7. Utilisation suivant la revendication 6, dans laquelle le polynucléotide comprend en outre une séquence de polyadénylation connectée de manière fonctionnelle à la séquence qui code pour le polypeptide VEGF-C.

8. Utilisation suivant l'une quelconque des revendications 1 à 7, dans laquelle la composition comprend un vecteur de thérapie génique, ledit vecteur de thérapie génique comprenant ledit polynucléotide, ledit vecteur comprenant facultativement un adénovirus à réplication déficiente, ledit adénovirus comprenant le polynucléotide connecté de manière fonctionnelle à un promoteur et flanqué par des séquences polynucléotidiques adénovirales.

9. Utilisation suivant l'une quelconque des revendications 1 à 8, dans laquelle la composition comprend en outre un support pharmaceutiquement acceptable.

10. Utilisation suivant l'une quelconque des revendications 1 à 9, dans laquelle le médicament est formulé pour l'administration :
(a) en au moins une injection intravasculaire ; ou
(b) en un transfert à médiation par cathéter de ladite composition dans un vaisseau sanguin du sujet mammifère, de préférence par introduction d'un cathéter dans une artère coronaire du sujet mammifère, et libération de la composition dans l'artère opposée.

11. Utilisation suivant l'une quelconque des revendications 1 à 10, dans laquelle le médicament est formulé pour l'administration conjointe, audit être humain, avec une angioplastie coronarienne transluminale percutanée.

12. Utilisation suivant la revendication 10 ou 11, dans laquelle le médicament est présent sous forme de revêtement sur un extenseur intravasculaire ou bien imprègne un extenseur intravasculaire.

13. Utilisation d'une composition dans la production d'un médicament destiné à prévenir la resténose d'un vaisseau sanguin chez un être humain, dans laquelle la composition comprend un vecteur consistant en adénovirus à réplication déficiente qui comprend un polynucléotide codant pour au moins un polypeptide choisi dans le groupe consistant en les polypeptides VEGF-C et les polypeptides VEGF-D, et comprenant en outre une séquence de promoteur servant de promoteur de l'expression dudit au moins un polypeptide dans les cellules du vaisseau sanguin.

14. Utilisation suivant la revendication 15, dans laquelle le polynucléotide code pour un polypeptide VEGF-C.

15. Extenseur endovasculaire conçu pour entrer en contact avec une surface d'un vaisseau sanguin au cours d'une intervention chirurgicale afin de traiter la sténose du vaisseau sanguin, l'extenseur comprenant une surface extérieure pour la mise en contact avec une surface d'un vaisseau sanguin, et une composition sur ladite surface, comprenant une composition suivant l'une quelconque des revendications 1 à 15.

16. Collier extravasculaire conçu pour la mise en contact avec une surface d'un vaisseau sanguin au cours d'une intervention chirurgicale pour traiter la sténose du vaisseau sanguin, le collier comprenant une paroi extérieure façonnée pour entourer la surface extérieure d'un vaisseau sanguin, la paroi entourant un espace contenant une composition suivant l'une quelconque des revendications 1 à 15.

17. Kit pour le traitement d'une resténose, comprenant un récipient renfermant une composition telle que définie dans la revendication 1 ou la revendication 14, une étiquette fixée au ou emballée avec le récipient, l'étiquette décrivant l'utilisation de la composition pour la prévention de la resténose d'un vaisseau sanguin, et :
(a) un dispositif médical choisi dans le groupe consistant en : des extenseurs intravasculaires, des cathéters intravasculaires, des colliers extravasculaires et des membranes adaptées à couvrir une surface d'un extenseur ou cathéter intravasculaire ; et/ou
(b) une substance de support pour délivrer l'agent à la paroi luminale d'un vaisseau, le support étant de préférence choisi dans le groupe consistant en des polymères sous forme d'hydrogels et des polymères en microparticules.
